(19) Europäisches Patentamt / European Patent Office / Office européen des brevets

(11) **EP 3 503 193 B1**

(12) **FASCICULE DE BREVET EUROPEEN**

(45) Date de publication et mention
de la délivrance du brevet:
**12.08.2020 Bulletin 2020/33**

(51) Int Cl.:
**H01L 27/146** *(2006.01)* **G01N 15/14** *(2006.01)*

(21) Numéro de dépôt: **18213235.7**

(22) Date de dépôt: **17.12.2018**

(54) **CAPTEUR D'IMAGE, PERMETTANT D'OBTENIR UNE INFORMATION RELATIVE À LA PHASE D'UNE ONDE LUMINEUSE**

BILDERFASSUNGSSENSOR, DER DEN EMPFANG EINER INFORMATION ÜBER EINE LICHTWELLENPHASE ERMÖGLICHT

IMAGE SENSOR, SUITABLE FOR OBTAINING INFORMATION ON THE PHASE OF A LIGHT WAVE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.12.2017 FR 1762492**

(43) Date de publication de la demande:
**26.06.2019 Bulletin 2019/26**

(73) Titulaire: **Commissariat à l'Énergie Atomique et aux Énergies Alternatives 75015 Paris cedex (FR)**

(72) Inventeurs:
• **BENAHMED, Selimen 38000 GRENOBLE (FR)**
• **BOUTAMI, Salim 38100 GRENOBLE (FR)**
• **CIONI, Olivier 38100 GRENOBLE (FR)**

(74) Mandataire: **GIE Innovation Competence Group 310, avenue Berthelot 69372 Lyon Cedex 08 (FR)**

(56) Documents cités:
WO-A1-2008/090330 WO-A1-2010/113634
US-A1- 2016 064 436

EP 3 503 193 B1

**Description**

## DOMAINE TECHNIQUE

Le domaine technique de l'invention est l'imagerie, et plus particulièrement l'imagerie de phase. L'invention est en particulier destinée à l'observation d'échantillons translucides ou transparents.

## ART ANTERIEUR

Lorsque l'on procède à l'observation d'échantillons transparents, ou faiblement opaques, les méthodes d'imagerie traditionnelles, basées sur l'utilisation d'un objectif, mis au point sur l'échantillon, ne sont généralement pas adaptées. On a développé des méthodes alternatives, permettant de former une image représentant la différence de phase entre une onde lumineuse ayant traversé l'échantillon et une onde lumineuse de référence, n'ayant pas interagi avec l'échantillon. C'est selon ce principe que des méthodes d'imagerie de phase, ou de contraste de phase ont été développées. Ces méthodes sont basées sur la formation d'interférences entre une onde lumineuse ayant interagi avec l'échantillon et l'onde lumineuse de référence. Cela permet de convertir une information relative à une différence de phase en une intensité, mesurable par un capteur d'image standard.

Mais de telles méthodes nécessitent des montages précis, et sont relativement complexes à mettre en œuvre pour des contrôles de routine, à faible coût. De plus, elles confèrent un champ d'observation relativement faible. Le document US 2016/064436 décrit un capteur d'image comportant une matrice de pixels.

Le document WO2008090330 décrit un dispositif permettant l'observation de particules biologiques translucides ou transparentes, en l'occurrence des cellules, par imagerie sans lentille. Le dispositif permet d'associer, à chaque cellule, une figure d'interférence dont la morphologie permet d'identifier le type de cellule. Il se base sur un capteur d'image simple, par exemple un capteur matriciel de type CCD ou CMOS. L'imagerie sans lentille apparaît alors comme une alternative simple, et peu onéreuse, à la microscopie de phase. De plus, elle permet d'obtenir un champ d'observation nettement plus étendu que ne peut l'être celui d'un microscope.

Cependant, l'image enregistrée par le capteur d'image ne comporte pas d'information relative à la phase. Pour obtenir une information représentative de la phase, il est nécessaire d'appliquer à l'image un opérateur de propagation holographique, de façon à reconstruire une image de phase, sur laquelle les contrastes de phase dus à l'échantillon apparaissent. Des exemples d'application d'opérateurs de propagation holographiques sont décrits dans les documents US2012/0218379, ou encore dans les documents WO2017162985 ou WO2016189257. Ces documents décrivent des algorithmes, le plus souvent itératifs, permettant d'obtenir progressivement une information relative à la phase d'un échantillon, tout en limitant le bruit de reconstruction. On parle d'algorithmes de reconstruction holographique, ou de reconstruction de phase.

De tels algorithmes sont d'autant plus performants que l'image acquise par le capteur d'image présente un rapport signal sur bruit élevé. A cette fin, le document EP3147646 décrit un support d'échantillon, destiné à être disposé face à un capteur d'image, et dans lequel est ménagé un réseau de diffraction monodimensionnel ou bidimensionnel. Le réseau de diffraction est dimensionné pour confiner une partie de l'onde lumineuse incidente dans un guide d'onde, s'étendant parallèlement au capteur d'image. Dans une bande spectrale, qui correspond à une bande spectrale de résonance du réseau de diffraction, le capteur d'image est placé en fond sombre. Le guide d'onde est disposé au contact de l'échantillon, de telle sorte que lorsqu'une particule de l'échantillon est disposée à proximité du guide d'onde, l'onde lumineuse confinée dans le guide d'onde est découplée localement, ce qui permet la formation d'un faisceau lumineux se propageant jusqu'au capteur d'image. Ce dispositif permet la formation de figures de diffraction avec un rapport signal sur bruit élevé. L'image formée peut ensuite faire l'objet d'un algorithme de reconstruction de phase.

Les inventeurs ont souhaité améliorer les performances des dispositifs existants d'imagerie sans lentille, en limitant, voire en évitant, le recours à des algorithmes de reconstruction de phase.

## EXPOSE DE L'INVENTION

Un premier objet de l'invention est un capteur d'image comportant une matrice de pixels, s'étendant selon un plan de détection, et configurée pour former une image d'une onde lumineuse incidente, se propageant, dans une bande spectrale, selon un axe de propagation, le capteur d'image étant caractérisé en ce qu'il comporte un masque, formé de différents masques élémentaires opaques, s'étendant parallèlement au plan de détection, entre lesquels s'étendent des ouvertures, à travers lesquelles l'onde lumineuse incidente est apte à se propager en direction du plan de détection, la matrice de pixels étant divisée en :

- des pixels ouverts s'étendant face aux ouvertures;
- des pixels masqués, chaque pixel masqué étant défini par une projection d'un masque élémentaire selon l'axe de propagation sur la matrice de pixels, chaque pixel masqué étant associé au masque élémentaire lui faisant face ;

le capteur d'image comportant, entre les pixels ouverts et les ouvertures:

- un guide d'onde, formant une bande s'étendant face à des pixels masqués et à des pixels ouverts ;
- un premier réseau de diffraction, s'étendant face à au moins un pixel ouvert, et configuré pour coupler une partie de l'onde lumineuse incidente dans le guide d'onde, de manière à former une onde guidée, le premier réseau de diffraction étant configuré pour transmettre une autre partie de l'onde lumineuse incidente vers le pixel ouvert, le premier réseau de diffraction étant associé au pixel ouvert
- un deuxième réseau de diffraction, s'étendant face à un pixel masqué, et configuré pour extraire une partie de l'onde guidée, se propageant dans le guide d'onde, de telle sorte que l'onde ainsi extraite se propage vers le pixel masqué, le deuxième réseau de diffraction étant associé au pixel masqué.

Selon un mode de réalisation,

- un pixel masqué s'étend entre deux pixels ouverts, qui lui sont adjacents, chaque pixel ouvert étant associé à un premier réseau de diffraction;
- le guide d'onde s'étend face aux deux pixels ouverts et face au pixel masqué;
- le pixel masqué est associé à un deuxième réseau de diffraction, de façon à extraire des ondes lumineuses guidées dans le guide d'onde, résultant respectivement d'un couplage de l'onde lumineuse incidente par le premier réseau de diffraction associé à chaque pixel ouvert adjacent du pixel masqué.

Le pixel masqué et les pixels ouverts lui étant adjacents peuvent être disposés selon une même ligne ou selon une même colonne de la matrice de pixels.

Selon un mode de réalisation,

- un pixel masqué s'étend entre deux pixels ouverts, qui lui sont adjacents, selon une ligne de la matrice de pixels, chaque pixel ouvert étant associé à un premier réseau de diffraction;
- le guide d'onde s'étend face aux deux pixels ouverts et face au pixel masqué, parallèlement à la ligne, formant un guide d'onde longitudinal, chaque premier réseau de diffraction associé aux pixels ouverts de la ligne étant configuré pour coupler une partie de l'onde lumineuse incidente dans le guide d'onde longitudinal;
- le pixel masqué est associé à un deuxième réseau de diffraction, de façon à extraire des ondes lumineuses guidées dans le guide d'onde longitudinal, de telle sorte que les ondes lumineuses ainsi extraites soient détectées par le pixel masqué;
- le pixel masqué s'étend entre deux pixels ouverts qui lui sont adjacents, selon une colonne de la matrice de pixels, lesdits pixels ouverts de la colonne étant associés à un premier réseau de diffraction;
- le capteur comporte un guide d'onde, dit guide d'onde latéral, s'étendant parallèlement à la colonne, face aux deux pixels ouverts de la colonne et face au pixel masqué, chaque premier réseau de diffraction associé aux pixels ouverts de la colonne étant configuré pour coupler une partie de l'onde lumineuse incidente dans le guide d'onde latéral;
- le deuxième réseau de diffraction est configuré pour extraire des ondes lumineuses se propageant dans le guide d'onde latéral, de telle sorte que les ondes lumineuses ainsi extraites soient détectées par le pixel masqué.

Selon un mode de réalisation,

- le premier réseau de diffraction est formé par une première couche mince, s'étendant parallèlement au guide d'onde, et formée d'un premier matériau, la première couche mince comportant des inclusions d'un premier matériau auxiliaire, les indices de réfraction respectifs du premier matériau et du premier matériau auxiliaire étant différents, le premier matériau et le premier matériau auxiliaire étant transparents dans tout ou partie de la bande spectrale ;
- le deuxième réseau de diffraction est formé par une deuxième couche mince, s'étendant parallèlement au guide d'onde, formée d'un deuxième matériau, la deuxième couche mince comportant des inclusions selon un deuxième matériau auxiliaire, les indices de réfraction respectifs du deuxième matériau et du deuxième matériau auxiliaire étant différents.

Le premier réseau de diffraction et/ou le deuxième réseau de diffraction peuvent être monodimensionnels ou bidimensionnels.

- Chaque premier réseau de diffraction peut former un réseau d'injection, pour injecter une partie de l'onde lumineuse incidente dans un guide d'onde s'étendant selon un axe. Dans ce cas, chaque premier réseau de diffraction peut être monodimensionnel.

- Chaque premier réseau de diffraction peut former un réseau d'injection, pour injecter une partie de l'onde lumineuse incidente dans deux guides d'ondes s'étendant respectivement selon des axes différents, notamment orthogonaux. Dans ce cas, chaque premier réseau de diffraction peut être bidimensionnel.

- Chaque deuxième réseau de diffraction peut former un réseau d'extraction, pour extraire une partie d'une l'onde lumineuse se propageant dans un guide d'onde s'étendant selon un axe. Dans ce cas, chaque deuxième réseau de diffraction peut être monodimensionnel.

- Chaque deuxième réseau de diffraction peut former un réseau d'extraction, pour extraire une partie d'une l'onde lumineuse se propageant dans des guides d'onde s'étendant respectivement selon des axes différents, notamment orthogonaux. Dans ce cas, chaque deuxième réseau de diffraction peut être bidimensionnel.

Selon un mode de réalisation le premier matériau et le deuxième matériau forment un même matériau. Il est de préférence transparent à la bande spectrale de l'onde lumineuse incidente. De préférence, le deuxième matériau auxiliaire est un métal.

Selon un mode de réalisation le premier réseau de diffraction et le deuxième réseau de diffraction s'étendent selon un même plan, de préférence orthogonal ou sensiblement orthogonal à l'axe de propagation.

Selon un mode de réalisation le premier réseau de diffraction et le deuxième réseau de diffraction s'étendent parallèlement à la matrice de pixels.

Selon un mode de réalisation au moins un masque élémentaire comporte une face réfléchissante, faisant face à un pixel masqué associé au masque élémentaire, de façon à renvoyer une partie d'onde lumineuse, extraite du deuxième guide d'onde s'étendant face au pixel masqué, vers ce dernier.

Un deuxième objet de l'invention est un dispositif d'observation d'un échantillon, comportant :

- une source de lumière, configurée pour émettre une onde lumineuse, dite onde lumineuse d'illumination, selon une bande spectrale, selon un axe de propagation, vers l'échantillon ;
- un capteur d'image;
- un support, apte à recevoir l'échantillon, de telle sorte que l'échantillon s'étend entre la source de lumière et l'échantillon, le capteur d'image étant configuré pour détecter une onde lumineuse incidente se propageant entre l'échantillon et le capteur lorsque l'échantillon est illuminé par l'onde lumineuse d'illumination ;

le capteur d'image étant un capteur selon le premier objet de l'invention.

Un troisième objet de l'invention est un procédé de détermination d'une intensité et d'une phase d'une onde lumineuse, en utilisant un capteur d'image selon le premier objet de l'invention, le capteur d'image étant tel que :

- un pixel masqué s'étend, selon une ligne ou une colonne, entre deux pixels ouverts ;
- le guide d'onde s'étend, face aux pixels ouverts et face au pixel masqué, selon ladite ligne ou colonne ;
- les pixels ouverts sont chacun associés à un premier réseau de diffraction;
- le pixel masqué est associé à un deuxième réseau de diffraction ;

le procédé comportant les étapes suivantes :

a) illumination du capteur de telle sorte qu'une onde lumineuse incidente se propage vers chaque pixel ouvert;
b) couplage d'une partie de l'onde lumineuse incidente le premier réseau de diffraction s'étendant face à chaque pixel ouvert, de façon à former, au niveau du premier réseau de diffraction, une onde lumineuse confinée se propageant dans le guide d'onde, et à transmettre, au pixel ouvert associé au premier réseau de diffraction, une onde lumineuse dite transmise;
c) extraction, par le deuxième réseau de diffraction, d'une partie de chaque onde lumineuse confinée, se propageant dans le guide d'onde, de façon à former une onde lumineuse extraite;
d) détection de l'onde lumineuse extraite par le pixel masqué;
e) à partir d'une intensité de de l'onde lumineuse extraite, obtention d'une information relative à différence de phase de l'onde lumineuse incidente se propageant respectivement vers chaque pixel ouvert;
f) détection l'onde lumineuse transmise par le premier réseau de diffraction, vers chaque pixel ouvert associé audit premier réseau de diffraction, de façon à obtenir une intensité de l'onde lumineuse transmise, représentative de l'intensité de l'onde lumineuse incidente au pixel ouvert.

Le procédé peut comporter une étape :

g) à partir de la différence de phases obtenue lors de l'étape e), estimation d'une phase de l'onde lumineuse détectée par chaque pixel ouvert.

Selon un mode de réalisation, les pixels ouverts sont disposés selon une même ligne ou une même colonne de la matrice de pixels, l'étape g) comportant une prise en compte d'une valeur de phase, dite de référence, obtenue sur un pixel de la ligne ou de la colonne.

Selon un mode de réalisation, dans lequel le capteur comporte différents pixels masqués, chaque pixel masqué étant disposé entre au moins deux pixels ouverts, selon une ligne, et entre au moins deux pixels ouverts, selon une colonne, les étapes a) à f) étant mises en œuvre sur chaque pixel masqué ainsi que sur les pixels ouverts entre lesquels le pixel masqué est disposé, l'étape g) étant réalisée à partir d'une différence de phase déterminée à partir d'une intensité d'une onde lumineuse détectée par chaque pixel masqué.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

La figure 1A représente un dispositif d'observation d'un échantillon selon l'invention.

La figure 1B montre une variante d'un capteur selon l'invention.

La figure 1C représente une vue d'un capteur d'image schématisé sur la figure 1A.

La figure 1D montre une évolution de l'intensité mesurée par un pixel masqué en fonction d'une différence de phase d'une onde lumineuse se propageant vers les pixels ouverts adjacents au pixel masqué.

Les figures 2A et 2B illustrent une autre mode de réalisation d'un capteur d'image selon l'invention. La figure 2C montre un autre mode de réalisation d'un capteur d'image selon l'invention.

Les figures 3A à 3L montrent différentes étapes de fabrication d'un capteur d'image tel que représenté sur la figure 1A.

Les figures 4A et 4B montrent des images de l'absorption et de la phase d'un échantillon surfacique modélisé pour effectuer des simulations.

Les figures 4C et 4D représentent respectivement des images du module et de la phase d'une onde lumineuse ayant traversé l'échantillon représenté sur la figure 4A et 4B et atteignant le plan de détection défini par le capteur d'image.

La figure 4E est obtenue à partir de la figure 4D. Elle représente des différences de phase entre deux pixels adjacents d'une même ligne.

Les figures 4F et 4G sont des résultats de reconstructions respectives du module et de la phase de l'onde lumineuse à partir de l'image de la figure 4C.

La figure 4H correspond à une image de la phase de l'onde lumineuse incidente au capteur, dans le plan de détection. Cette image est obtenue à partir des différences de phases représentées sur la figure 4E en utilisant, sur chaque ligne, des valeurs de phase, dites de référence, obtenues sur la figure 4G.

Les figures 4I et 4J sont des images du module et de la phase de l'onde lumineuse incidente au capteur, dans le plan de l'échantillon. Ces images sont obtenues par une reconstruction holographique effectuée à partir des images 4F et 4G.

## EXPOSE DE MODES DE REALISATION PARTICULIERS

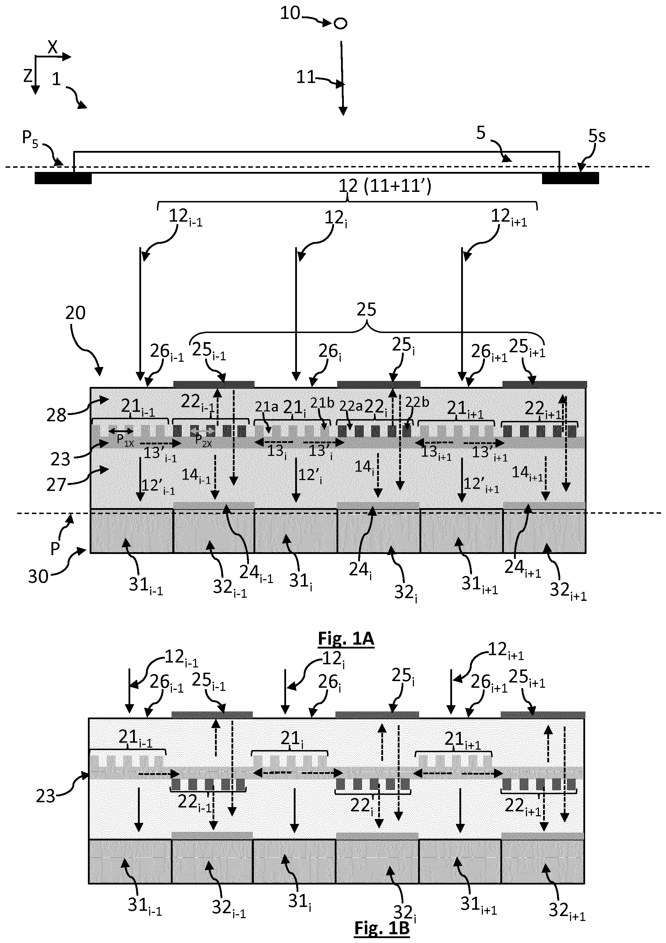

On a représenté, sur la figure 1A, un dispositif d'observation d'un échantillon selon l'invention. Le dispositif 1 comporte une source de lumière 10, configurée pour illuminer un échantillon 5. La source de lumière 10 est configurée pour émettre une onde lumineuse d'illumination 11 se propageant vers l'échantillon selon un axe de propagation Z, dans une bande spectrale $\Delta\lambda$.

L'échantillon 5 est un échantillon transparent ou translucide que l'on souhaite caractériser. Il peut par exemple s'agir d'un liquide, comportant des particules. Le liquide peut comporter un fluide biologique, par exemple du sang ou un extrait de sang, ou tout autre liquide, par exemple de la lymphe, de l'urine, du liquide céphalo rachidien... Le liquide peut également être un milieu de culture cellulaire, ou un liquide prélevé dans l'environnement ou dans une installation industrielle. Les particules peuvent être des cellules, des microorganismes, par exemple des bactéries ou des levures ou des spores. Il peut également s'agir de microbilles, ou de particules lipidiques, ou des particules solides. L'échantillon peut également être solide, ou prendre la forme d'un gel. Il peut par exemple s'agir d'une fine lame de tissu que l'on souhaite caractériser, par exemple une lame d'anatomo-pathologie. L'échantillon est disposé sur un support d'échantillon 5s. Le support d'échantillon définit un plan d'échantillon $P_5$, de telle sorte que lorsque l'échantillon est disposé sur le support, il s'étend selon le plan d'échantillon $P_5$. Le plan d'échantillon $P_5$ est de préférence orthogonal, ou sensiblement orthogonal, à l'axe de propagation Z de l'onde lumineuse d'illumination 11. Par sensiblement orthogonal, on entend formant un angle de 90° à une tolérance angulaire près, par exemple ± 10° ou ± 20°. L'échantillon peut être disposé dans une chambre fluidique, ou tout autre récipient transparent, ou sur une lame transparente. L'épaisseur de l'échantillon, selon l'axe de propagation Z, est de préférence inférieure à 1 cm, et est généralement inférieure à 5 mm ou 1 mm.

La source de lumière 10 peut être une source de lumière laser ou une source de lumière de type diode électroluminescente. Elle peut être couplée à une filtre passe bande, non représenté sur la figure 1A, de façon à limiter la bande spectrale $\Delta\lambda$ de l'onde lumineuse 11 atteignant l'échantillon 5. En effet, comme décrit par la suite, le dispositif 1 comporte un capteur d'image 20, comportant des réseaux de diffraction dont les caractéristiques sont adaptées à une longueur d'onde de résonance $\lambda$. La bande spectrale $\Delta\lambda$ doit donc s'étendre autour de la longueur d'onde de résonance $\lambda$ décrite par la suite. La largeur de la bande spectrale $\Delta\lambda$ de l'onde lumineuse d'illumination 11 est de préférence inférieure à 100 nm, voire inférieure à 50 nm ou à 10 nm, de telle sorte que l'onde lumineuse 11 puisse être assimilée à une onde monochromatique. Par largeur de bande, on entend une largeur à mi-hauteur de la bande spectrale. De préférence, la source de lumière est ponctuelle, de telle sorte que l'onde lumineuse atteint l'échantillon 5 sous la forme d'une onde plane, se propageant selon l'axe de propagation Z. La source de lumière 10 peut être associée à un diaphragme. Lorsque la source de lumière comporte un diaphragme, un diffuseur est de préférence être inséré entre la source de lumière et le diaphragme, comme décrit dans le document US2017317125. La source de lumière 10 peut également être formée par une extrémité d'une fibre optique, dont une autre extrémité est couplée à une source lumineuse. Il peut en particulier s'agir d'une fibre optique monomode.

Une partie de l'onde lumineuse d'illumination 11 est transmise par l'échantillon 5, sans interagir, ou de façon négligeable, avec ce dernier. Une autre partie de l'onde lumineuse 11 interagit avec l'échantillon, et est par exemple diffractée par ce dernier.

Le dispositif comporte un capteur d'image 20. Dans l'exemple représenté sur la figure 1A, l'échantillon 5 est disposé entre la source de lumière 10 et le capteur d'image 20. Le capteur d'image 20 reçoit une onde lumineuse 12, dite onde lumineuse incidente, comportant :

- une partie de l'onde lumineuse d'illumination 11 transmise par l'échantillon ;
- une onde lumineuse diffractée 11', issue de la diffraction, par l'échantillon, de l'onde lumineuse d'illumination 11.

L'onde lumineuse incidente 12, à laquelle est exposé le capteur d'image 20, se propage selon l'axe de propagation Z vers le capteur d'image.

Dans l'exemple représenté sur la figure 1A, aucun système optique de formation d'image n'est disposé entre l'échantillon 5 et le capteur d'image 20. Ainsi, le capteur d'image est configuré selon une configuration dite d'imagerie sans lentille.

Dans les procédés de l'art antérieur, on détecte, à l'aide d'un capteur d'image standard, l'onde lumineuse incidente 12. Puis, au moyen d'algorithmes de reconstruction holographique, on détermine une phase de l'onde lumineuse 12, en particulier dans le plan de l'échantillon $P_5$. Le capteur d'image 20 décrit ci-après limite le recours à de tels algorithmes de reconstruction. Il se distingue de l'art antérieur en ce qu'il permet d'accéder à une information de phase de l'onde lumineuse incidente 12, comme décrit par la suite.

De façon classique, le capteur d'image 20 comporte une matrice de pixels 30, s'étendant selon un plan P formant un plan de détection. Les pixels sont formés dans un substrat de silicium, selon une technologie CMOS. Le plan de détection P est de préférence orthogonal, ou sensiblement orthogonal, à l'axe de propagation Z.

Le capteur d'image comporte un masque 25, disposé en amont de la matrice de pixels 30. Le terme amont étant à comprendre selon le sens propagation de la lumière. Le masque 25 comporte des masques élémentaires $25_i$ opaques dans la bande spectrale $\Delta\lambda$. Chaque masque élémentaire $25_i$ s'étend de préférence parallèlement à la matrice de pixels 30. L'indice i est un entier correspondant à une position du masque élémentaire perpendiculairement à l'axe Z. En considérant la direction de propagation Z, chaque masque élémentaire $25_i$ se projette sur la matrice de pixels 30, de façon à former, sur cette dernière, des pixels dits masqués $32_i$. Sur l'exemple représenté, la taille d'un masque élémentaire $25_i$ correspond à la taille d'un pixel, cette dernière étant de 1 $\mu$m x 1 $\mu$m. D'une façon plus générale, la taille de chaque pixel est de préférence inférieure à 10 $\mu$m x 10 $\mu$m.

Ainsi, à chaque masque élémentaire $25_i$ est associé un pixel masqué $32_i$, le pixel masqué s'étendant face au masque élémentaire $25_i$ auquel il est associé, selon l'axe de propagation Z. Chaque masque élémentaire $25_i$ est, dans cet exemple, formé d'une couche d'aluminium d'épaisseur, selon l'axe Z, égale à 100 nm. Un masque élémentaire $25_i$ peut être réalisé en utilisant un autre matériau, sous réserve que le matériau soit opaque dans la bande spectrale $\Delta\lambda$. On a représenté, sur la figure 1A, trois masques élémentaires $25_{i-1}$, $25_i$, et $25_{i+1}$.

Entre deux masques élémentaires adjacents $25_{i-1}$, $25_i$ s'étendent des ouvertures $26_i$. Chaque ouverture est transparente dans la bande spectrale $\Delta\lambda$. On a représenté, sur la figure 1A, une ouverture $26_i$ s'étendant entre deux masques élémentaires adjacents $25_{i-1}$ et $25_i$, ainsi que deux autres ouvertures $26_{i-1}$ et $26_{i+1}$. La dimension d'une ouverture, parallèlement au plan de détection P, correspond de préférence à la dimension d'au moins un pixel de la matrice de pixels 30. Ainsi, de façon analogue aux masques élémentaires $25_i$, chaque ouverture $26_i$ se projette, selon l'axe de propagation Z, sur la matrice de pixel 30, en formant un pixel $31_i$, dit pixel ouvert. Ainsi, à chaque pixel ouvert $31_i$ est associée une ouverture $26_i$, cette dernière se situant face au pixel ouvert $31_i$, selon l'axe de propagation Z. A chaque pixel ouvert $31_i$ est associée une ouverture $26_i$, disposée face à ce dernier, selon l'axe de propagation Z. Une ouverture $26_i$ peut être formée d'un matériau transparent ou d'un espace laissé libre, adjacent d'un masque élémentaire $25_i$. Sur la figure 1A, on a représenté des ondes lumineuses $12_{i-1}$, $12_i$ et $12_{i+1}$ se propageant respectivement à travers les ouvertures $26_{i-1}$, $26_i$ et $26_{i+1}$ vers les pixels ouverts $31_{i-1}$, $31_i$ et $31_{i+1}$. Les ondes lumineuses $12_{i-1}$, $12_i$ et $12_{i+1}$ forment l'onde lumineuse incidente 12 à laquelle est exposé le capteur d'image 20.

Dans les exemples décrits ci-après, le masque est arrangé en formant un damier, s'étendant parallèlement au plan de détection P. Les pixels de la matrice de pixels sont arrangés en lignes et en colonnes. Chaque ligne et chaque colonne comprend des pixels ouverts $31_{i-1}$, $31_i$ et $31_{i+1}$ et des pixels masqués $32_{i-1}$, $32_i$ et $32_{i+1}$ disposés en alternance.

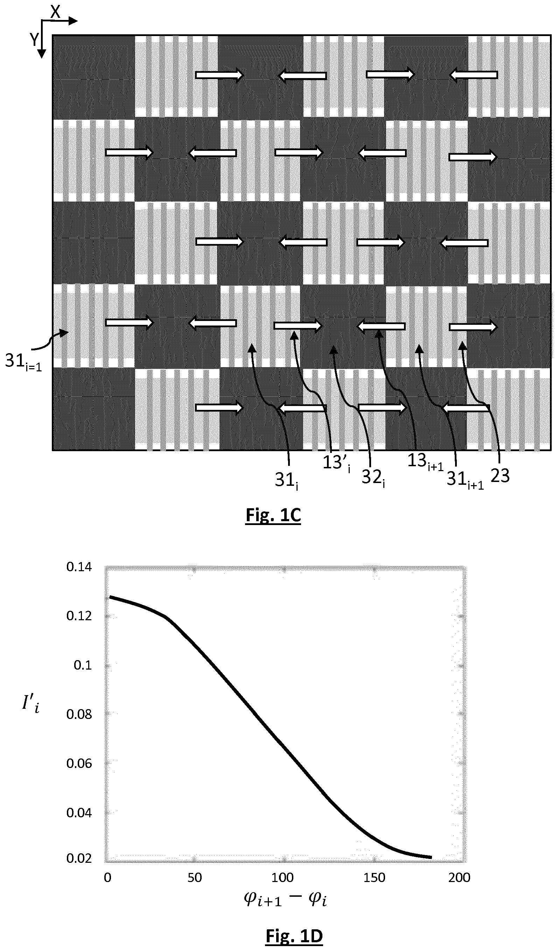

La figure 1C représente une vue de dessus d'un capteur d'image tel que représenté sur la figure 1A. On observe la disposition des masques élémentaires $25_i$ et des ouvertures $26_i$ sous la forme d'un damier. Sous chaque masque élémentaire $25_i$ est disposé un pixel masqué $32_i$. Sous chaque ouverture est disposé un pixel ouvert $31_i$. A l'exception des pixels disposés à la périphérie de la matrice, la plupart des pixels masqués $32_i$ sont disposés entre deux pixels ouverts $31_{i-1}$, $31_i$, dits pixels ouverts adjacents, selon une même ligne.

Sur la figure 1A, on a représenté des pixels appartenant à une même ligne, s'étendant selon un axe longitudinal X. Un guide d'onde longitudinal 23 s'étend parallèlement à chaque ligne de pixels selon l'axe longitudinal X. Selon un axe latéral Y, perpendiculaire à l'axe longitudinal X, le guide d'onde longitudinal 23 a une largeur inférieure ou égale à la largeur des pixels. Les axes X et Y sont coplanaires au plan de détection P. Le guide d'onde 23 est de préférence centré par rapport à la ligne de pixels face à laquelle il s'étend. Le guide d'onde forme une bande s'étendant face à plusieurs pixels d'une même ligne, tout en ayant une largeur inférieure ou égale à la largeur des pixels formant la ligne. Il prend ainsi une forme de bande s'étendant parallèlement au plan de détection P. L'indice de réfraction du matériau formant le guide d'onde 23 est supérieur à l'indice de réfraction des matériaux desquels il est adjacent.

Entre chaque pixel ouvert $31_i$ de la ligne de pixels et l'ouverture $26_i$ associé audit pixel, un premier réseau de diffraction $21_i$ est ménagé. Par réseau de diffraction, on entend une structure dont l'indice de réfraction varie périodiquement à l'échelle de la longueur d'onde $\lambda$, selon une ou plusieurs directions. Le premier réseau de diffraction est ménagé $21_i$ dans une couche mince, dite première couche mince, formée d'un premier matériau 21a, dans laquelle sont disposées, de façon périodique, des inclusions d'un premier matériau auxiliaire 21b. Le premier matériau auxiliaire 21b est différent du premier matériau 21a. De préférence, le premier matériau 21a et le premier matériau auxiliaire 21b sont des matériaux diélectriques, transparents dans tout ou partie de la bande spectrale $\Delta\lambda$ de l'onde lumineuse incidente $12_i$ se propageant à travers l'ouverture $26_i$. Les indices de réfraction respectifs du premier matériau 21a et du premier matériau auxiliaire 21b sont différents. Dans l'exemple décrit, le premier matériau 21a formant la couche mince est du dioxyde de silicium ($SiO_2$), tandis que le premier matériau auxiliaire 21b formant les inclusions est du nitrure de silicium (SiN). La première couche mince a dans cet exemple une épaisseur de 50 nm, selon l'axe Z.

Les inclusions, visibles sur les figures 1A, 1B et 1C, définissent un motif spatial périodique de période $P_1x$ = 240 nm. Le premier réseau de diffraction $21_i$ est un réseau de diffraction monodimensionnel, dans le sens où sa périodicité est définie dans une seule dimension, en l'occurrence selon l'axe X. Sur les figures 1A et 1B, on a représenté un premier réseau de diffraction $21_{i-1}$, $21_i$, $21_{i+1}$ respectivement associé aux pixels ouverts $31_{i-1}$, $31_i$ et $31_{i+1}$.

Les inclusions du premier matériau auxiliaire 21b ont ici une faible longueur selon l'axe longitudinal X, de l'ordre de 80 à 160 mm et s'étendent selon tout ou partie de la largeur du pixel selon l'axe latéral Y, par exemple selon 80% à 100% de cette largeur.

Le réseau de diffraction $21_i$ agit en tant que réseau de couplage pour coupler une partie de l'onde lumineuse incidente $12_i$, se propageant vers le pixel ouvert $31_i$ selon l'axe Z, dans le guide d'onde longitudinal 23. Un tel couplage est connu en soi, et a par exemple été décrit dans EP3147646, ou dans des publications, par exemple la publication Taillaert D, "Compact efficient broadband grating coupler for silicon-on-insulator waveguides", Optics Letters, Vol. 29, N° 23, december 1, 2004. Le matériau formant le guide d'onde longitudinal 23 est dans cet exemple du nitrure de silicium. Il correspond alors au premier matériau auxiliaire 21b du premier réseau de diffraction. Il pourrait s'agir d'un autre matériau, sous réserve que son indice de réfraction soit supérieur à l'indice de réfraction du premier matériau 21a ainsi qu'à l'indice de réfraction du matériau d'une couche inférieure 27, cette dernière s'étendant entre la matrice de pixels et le guide d'onde 23. L'épaisseur du guide d'onde 23, selon l'axe Z, est de 100 nm.

Dans cet exemple, le matériau formant la couche inférieure 27 est du dioxyde de silicium ($SiO_2$). L'épaisseur de la couche inférieure 27, selon l'axe Z, est de 170 nm.

D'une façon générale, la période $P_1x$ du motif formant le premier réseau de diffraction correspond à la longueur d'onde $\lambda$ de résonance du premier réseau de diffraction divisée par un indice optique effectif $n_{eff}$, de telle sorte que

$$P_{1,X} = \frac{\lambda}{n_{eff}}$$

$n_{eff}$ est compris entre l'indice de réfraction du premier matériau 21a, en l'occurrence la silice ($SiO_2$) et l'indice de réfraction du matériau formant le guide d'onde 23.

Lorsque l'onde incidente $12_i$ se propage, à travers une ouverture $26_i$, vers un pixel ouvert $31_i$, faisant face à l'ouverture, une partie de l'onde incidente $12_i$ est couplée, par le premier réseau de diffraction $21_i$, dans le guide d'onde 23. Elle forme alors une onde confinée, se propageant dans le guide d'onde. Le premier réseau de diffraction $21_i$ forme alors un réseau d'injection, car il injecte une partie de l'onde lumineuse incidente $12_i$ dans le guide d'onde 23. Compte tenu de l'angle d'incidence de l'onde lumineuse incidente $12_i$, orthogonal ou sensiblement orthogonal au plan selon lequel s'étend le premier réseau de diffraction $21_i$, le couplage dans le premier réseau de diffraction donne naissance à :

- une onde confinée $13_i$ se propageant selon un sens de propagation, dans le guide d'onde 23, vers un pixel masqué $32_{i-1}$ ;
- une onde confinée $13'_i$, se propageant dans le sens opposé à l'onde confinée $13_i$, vers un pixel masqué $32_i$.

Ainsi, environ 50% de l'onde incidente couplée dans le guide d'onde 23 se propage selon un sens, tandis que 50% de l'onde incidente couplée se propage dans le guide d'onde selon un sens opposé. Selon les deux sens de propagation, les ondes confinées $13_i$ et $13'_i$ ont la même amplitude et la même phase.

La partie de l'onde lumineuse incidente $12'_i$, non couplée par le premier réseau de diffraction $21_i$, est transmise par ce dernier et se propage vers le pixel ouvert $31_i$, où son intensité peut être mesurée. Ainsi, chaque pixel ouvert $31_i$ forme un pixel "d'intensité", car étant apte à mesurer une intensité de l'onde lumineuse incidente $12_i$. On considère que 25% de l'onde incidente $12_i$ peut être couplée dans le guide d'onde 23, ce pourcentage pouvant être inférieur.

Sur la figure 1A, on a également représentées des ondes couplées $13_{i+1}$ et $13'_{i+1}$, résultant du couplage de l'onde incidente $12_{i+1}$ avec le premier réseau de diffraction $21_{i+1}$, et se propageant respectivement dans le guide d'onde 23 vers les pixels masqués $32_i$ et $32_{i+1}$. On a également représenté une onde couplée $13'_{i-1}$ résultant du couplage de l'onde incidente $12_{i-1}$ avec le premier réseau de diffraction $21_{i-1}$, et se propageant respectivement dans le guide d'onde 23 vers le pixel masqué $32_{i-1}$. On a également représenté des ondes lumineuses $12'_{i-1}$ et $12'_{i+1}$ respectivement transmises, par les premiers réseaux de diffractions $21_{i-1}$ et $21_{i+1}$, vers les pixels ouverts $31_{i-1}$ et $31_{i+1}$.

Le capteur d'image 20 comporte un deuxième réseau de diffraction $22_i$ s'étendant au niveau de chaque pixel masqué $32_i$. Le deuxième réseau de diffraction $22_i$ est ménagé dans une couche mince, dite deuxième couche mince, formée d'un deuxième matériau 22a, dans laquelle sont disposées des inclusions d'un deuxième matériau auxiliaire 22b, différent du premier matériau 22a. Dans cet exemple, la deuxième couche mince est formée du même matériau que la première couche mince, formant le premier réseau de diffraction, à savoir $SiO_2$. Ainsi, le deuxième matériau 22a est $SiO_2$. Le deuxième matériau auxiliaire 22b peut être un matériau diélectrique, comme SiN, mais les inventeurs ont estimé qu'il était préférable que le deuxième matériau auxiliaire 22b soit un métal, par exemple de l'aluminium. Le recours à un métal permet d'augmenter le taux de découplage, c'est-à-dire le pourcentage de l'onde lumineuse extraite du guide d'onde. Un avantage de l'aluminium est qu'il est compatible avec la plupart des procédés de fabrication CMOS. La fonction du deuxième réseau de diffraction $22_i$ est d'extraire la lumière se propageant dans le guide d'onde 23. Ainsi :

- le premier réseau de diffraction $21_i$, associé à chaque pixel ouvert $31_i$, est un réseau d'injection, dimensionné pour injecter une partie de l'onde lumineuse incidente $12_i$ dans le guide d'onde 23 ;
- le deuxième réseau de diffraction $22_i$, associé à chaque pixel masqué $32_i$, est un réseau d'extraction, dimensionné pour extraire une onde lumineuse confinée se propageant dans le guide d'onde 23.

De préférence, le taux de couplage du premier réseau de diffraction $21_i$, c'est-à-dire le pourcentage d'onde lumineuse $12_i$ couplée dans le guide d'onde 23, est inférieur au taux de découplage du deuxième réseau de diffraction $22_i$.

Comme précédemment décrit en lien avec le premier réseau de diffraction $21_i$, les inclusions formant le deuxième réseau de diffraction sont périodiques, et s'étendent selon l'axe latéral Y, selon tout ou partie de la largeur du pixel, par exemple entre 80 % et 100% de la largeur du pixel. Leur épaisseur, selon l'axe Z, est de 50 nm. Dans cet exemple, les inclusions sont formées d'aluminium, selon un pas périodique $P_2x$ de 240 nm. La longueur de chaque inclusion, selon l'axe X, est comprise entre 80 et 160 nm. Sous l'effet du deuxième réseau de diffraction $22_i$, chaque onde lumineuse confinée se propageant dans le guide d'onde 23 est découplée, et forme une onde $14_i$ découplée se propageant vers le pixel masqué $32_i$. Sur la figure 1A, on a représenté des deuxièmes réseaux de diffractions $22_{i-1}$ et $22_{i+1}$ respectivement associés aux pixels masqués $32_{i-1}$ et $32_{i+1}$, ainsi que des ondes lumineuses découplées $14_{i-1}$ et $14_{i+1}$.

Avantageusement, chaque masque élémentaire $25_i$ comporte une face réfléchissante orientée vers le guide d'onde 23. Ainsi, une partie de l'onde découplée se propage vers le masque puis est réfléchie pour se propager vers

le pixel masqué $32_i$. Cela permet d'augmenter l'intensité du signal détecté par le pixel masqué $32_i$.

Le dimensionnement du premier et du deuxième réseau de diffraction, ainsi que la détermination de leur longueur d'onde de résonance, peuvent être effectués par des codes de calcul. En effet, les propriétés de propagation de la lumière dans des réseaux de diffraction découlent de leur arrangement périodique spécifique et peuvent être modélisées, par l'homme du métier, sur la base des équations spatio-temporelles de Maxwell. Dans le cas présent, les réseaux de diffraction ont été modélisés à l'aide du logiciel Rsoft, mettant en œuvre une méthode de différences finies dans le domaine temporel, désignée par l'acronyme FDTD (Finite Différence Time Domain). De préférence, les premier et deuxième réseaux de diffraction ont la même longueur d'onde de résonance. Dans cet exemple, la longueur d'onde de résonance $\lambda$ est de 405 nm.

Le capteur 20 comporte une couche supérieure 28, intercalée entre le premier ou le deuxième réseau de diffraction, et les ouvertures ou les masques élémentaires. La couche supérieure 28 est dans cet exemple formée de $SiO_2$, d'épaisseur 150 nm.

Une couche antireflet $24_i$ peut être disposée au niveau de chaque pixel, et notamment de chaque pixel masqué $32_i$. Il peut par exemple s'agir d'une couche en SiN, d'épaisseur 50 nm, soit d'épaisseur quart d'onde, égale à $\frac{\lambda}{4n_{SiN}}$, où $n_{SiN}$ désigne l'indice de réfraction du SiN. Une telle couche est optionnelle. Sur la figure 1A, on a également représenté des couches antireflet $24_{i-1}$ et $24_{i+1}$ respectivement associées aux pixels masqués $32_{i-1}$ et $32_{i+1}$.

La figure 1B représente une variante de la figure 1A, selon laquelle les deuxièmes réseau de diffraction $22_{i-1}$, $22_i$, $22_{i+1}$ sont disposés selon une couche mince s'étendant entre le guide d'onde 23 et les pixels. Selon ce mode de réalisation, les premiers réseaux de diffraction et les deuxièmes réseaux de diffraction sont disposés le long de deux faces opposées du guide d'onde 23.

Soient $I_i$ et $\varphi_i$, l'intensité et la phase de l'onde lumineuse $12_i$. Soient $I_{i+1}$ et $\varphi_{i+1}$, l'intensité et la phase de l'onde lumineuse $12_{i+1}$. Soit $2\tau$, le taux de couplage de chaque premier réseau de diffraction. Au niveau du premier réseau de diffraction $21_i$, une onde lumineuse confinée $13_i$ se forme selon un taux de couplage $\tau$, et il en est de même pour l'onde lumineuse confinée $13'_i$.

Ainsi, l'onde lumineuse $12'_i$ se propageant jusqu'au pixel ouvert $31_i$ a une amplitude $A_i$, telle que

$$A_i = (1 - 2\tau)a_i e^{j\varphi_i} \text{ (1)}$$

où j désigne l'unité imaginaire du nombre complexe ($j^2$ = -1) et $a_i$ est l'amplitude de l'onde lumineuse incidente $12_i$.

L'intensité lumineuse détectée par le pixel ouvert $31_i$ est

$$I_i = (1 - 2\tau)^2 a_i^2 \text{ (1')}$$

Le même raisonnement peut être effectué à l'égard du pixel ouvert $31_{i+1}$. Ainsi, l'onde lumineuse $12'_{i+1}$ se propageant jusqu'au pixel ouvert $31_{i+1}$ a une amplitude $A_{i+1}$ telle que

$$A_{i+1} = (1 - 2\tau)a_{i+1} e^{j\varphi_{i+1}} \text{ (2)}$$

où $a_{i+1}$ est l'amplitude de l'onde lumineuse incidente $12_{i+1}$.

L'intensité lumineuse détectée par le pixel ouvert $31_{i+1}$ est

$$I_{i+1} = (1 - 2\tau)^2 a_{i+1}^2 \text{ (2')}$$

Une partie de l'onde lumineuse incidente $12_i$ est couplée par le premier réseau de diffraction $21_i$ dans le guide d'onde 23. Elle forme une onde lumineuse guidée $13'_i$, se propageant en direction du deuxième réseau de diffraction $22_i$, avec une amplitude

$$A_i^* = \tau a_i e^{j\varphi_i} \text{ (3)}.$$

De même, une partie de l'onde lumineuse incidente $12_{i+1}$ est couplée par le premier réseau de diffraction $21_{i+1}$

dans le guide d'onde 23. Elle forme une onde lumineuse guidée $13_{i+1}$, se propageant en direction du deuxième réseau de diffraction $22_i$, avec une amplitude $A^*_{i+1} = \tau a_{i+1}e^{j\varphi_{i+1}}$ (4). (4).

Sous l'effet du découplage par le deuxième réseau de diffraction $22_i$, il se forme une onde lumineuse $14_i$ dont l'amplitude $A'_i$, en supposant un découplage total, et une réflexion totale par le masque $25_i$, est telle que :

$$A'_i = A^*_i + A^*_{i+1} = \tau a_i e^{j\varphi_i} + \tau a_{i+1}e^{j\varphi_{i+1}} \quad (5)$$

L'intensité $I'_i$ détectée par le pixel masqué $32_i$ est telle que :

$$I'_i = \left|\tau a_i e^{j\varphi_i} + \tau a_{i+1}e^{j\varphi_{i+1}}\right|^2 \quad (6)$$

Soit

$$I'_i = \tau^2\left(a_i^2 + a_{i+1}^2 + 2a_i a_{i+1}\cos(\varphi_{i+1} - \varphi_i)\right) \quad (7).$$

Les intensités $I_i$ et $I_{i+1}$ respectivement mesurées par les pixels ouverts $31_i$ et $31_{i+1}$ permettent d'estimer respectivement $a_i$ et $a_{i+1}$, selon les expressions (1') et (2').

Connaissant $a_i$ et $a_{i+1}$, l'intensité mesurée par le pixel masqué $32_i$ permet d'estimer $\cos(\varphi_{i+1}-\varphi_i)$, dont on peut déduire $\varphi_{i+1}-\varphi_i$.

Le taux de couplage $\tau$ est obtenu soit par construction, soit par des mesures expérimentales, en illuminant le capteur 20 avec une source de lumière de calibration, dont on maîtrise l'intensité, et sans échantillon 5 entre le capteur 20 et la source de lumière de calibration. Soit $a_c$, l'amplitude de la source de lumière de calibration, le déphasage de l'onde lumineuse entre les deux pixels ouverts $31_i$ et $31_{i+1}$ étant nul, on obtient, par application respective des expressions (1'), (2') et (7) :

$$I_{i,c} = (1 - 2\tau)^2 a_c^2 \ ;$$

$$I_{i+1,c} = (1 - 2\tau)^2 a_c^2 \ ;$$

$$I'_{i,c} = 4\tau^2 a_c^2;$$

$I_{i,c}$, $I_{i+1,c}$ et $I'_{i,c}$ étant les intensités respectivement mesurées par les pixels ouverts $31_i$, $31_{i+1}$ et le pixel masqué $32_i$ au cours de la calibration.

Le ratio $\dfrac{I'_{i,c}}{I_{i,c}}$ permet de déterminer $\tau$.

L'expression (7) montre que l'intensité mesurée par le pixel masqué $32_i$ dépend du déphasage des ondes lumineuses $12_i$, $12_{i+1}$ illuminant respectivement les pixels ouverts $31_i$ et $31_{i+1}$ adjacents. Autrement dit, l'intensité mesurée par le pixel masqué $32_i$ dépend d'une différence de phase entre les ondes lumineuses $12_i$ et $12_{i+1}$. Le pixel masqué $32_i$ permet ainsi d'accéder à une information relative au déphasage entre l'onde lumineuse incidente aux deux pixels ouverts $31_i$, $31_{i+1}$ qui lui sont adjacents. Il s'agit donc d'un pixel dit "de phase".

Sur la base de simulations, on a estimé l'intensité lumineuse d'un pixel masqué en fonction du déphasage des ondes lumineuses incidentes aux pixels ouverts qui lui sont adjacents. Le résultat de la simulation est représenté sur la figure 1D. L'axe des ordonnées représente l'intensité lumineuse détectée, tandis que l'axe des abscisses représente la différence de phase.

On constate que lorsque les ondes $12_i$ et $12_{i+1}$ sont en phase, l'intensité mesurée par le pixel masqué $32_i$ est maximale. Lorsque les ondes $12_i$ et $12_{i+1}$ sont en opposition de phase, l'intensité mesurée par le pixel masqué $32_i$ est minimale.

L'information de phase obtenue par chaque pixel masqué $32_i$ est une information différentielle, et représente une différence de phase $\varphi_{i+1} - \varphi_i$ entre les ondes lumineuses $12_{i+1}$ et $12_i$ incidentes aux pixels ouverts adjacents du pixel masqué. Selon ce mode de réalisation, comme on peut le voir sur la figure 1C, chaque pixel masqué $32_i$ permet d'obtenir une différence de phase entre les ondes lumineuses incidentes aux pixels ouverts, qui lui sont adjacents, selon une même ligne. Cela est représenté par les flèches blanches matérialisées sur la figure 1C, chaque flèche blanche représentant la propagation d'une onde guidée entre un pixel ouvert et un pixel masqué qui lui est adjacent. Si, sur au moins un pixel de chaque ligne, on obtient une phase connue, dite phase de référence $\varphi_{ref}$, la phase $\varphi_i$ de chaque onde lumineuse incidente à chaque pixel ouvert $31_i$ de la même ligne peut se déduire de proche en proche. Par exemple, si la phase $\varphi_1$ de l'onde parvenant au pixel $31_{i=1}$ situé sur la première colonne, est connue, la phase des ondes lumineuses incidentes aux autres pixels ouverts $31_i$ d'une même ligne se déduisent de proche en proche, connaissant les différences de phase $\varphi_{i+1} - \varphi_i$ respectivement mesurées par les pixels masqués $32_i$ de la ligne.

Aussi, d'une façon générale, le capteur 20 permet d'obtenir, par chaque pixel masqué $32_i$, une différence de phase des ondes lumineuses $12_i$, $12_{i+1}$ respectivement incidentes aux pixels ouverts $31_i$, $31_{i+1}$ qui lui sont adjacents, selon une même ligne. La prise en compte d'une phase de référence $\varphi_{ref}$, sur au moins un pixel de la ligne, permet d'obtenir une valeur de la phase des ondes lumineuses incidentes atteignant les pixels ouverts d'une même ligne. Selon ce mode de réalisation, où les pixels masqués de chaque ligne sont indépendants d'une ligne à l'autre, il est préférable de prendre en compte une phase de *référence* $\varphi_{ref}$ sur chaque ligne. Par pixels masqués indépendants, on entend le fait que les pixels masqués ne reçoivent pas une onde lumineuse guidée provenant d'une autre ligne.

La phase de référence $\varphi_{ref}$ peut être établie par un algorithme classique de reconstruction holographique, à partir de l'intensité lumineuse mesurée par un pixel ouvert, et cela pour chaque ligne. Par rapport à l'art antérieur, la reconstruction holographique peut se limiter à un seul pixel par ligne, et non à l'ensemble des pixels.

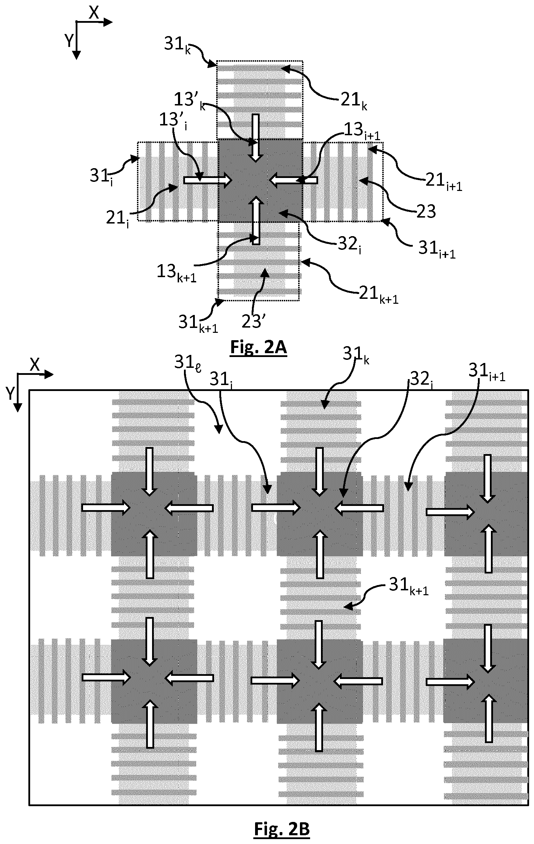

Dans le mode de réalisation décrit en lien avec les figures 2A, on a représenté un pixel masqué $32_i$, tel que précédemment décrit. Le pixel masqué $32_i$ est adjacent, selon une ligne, à deux pixels ouverts $31_i$ et $31_{i+1}$, similaires à ceux précédemment décrits. Le pixel masqué $32_i$ est également adjacent, selon une colonne, à deux pixels ouverts $31_k$ et $31_{k+1}$. Selon ce mode de réalisation :

- un guide d'onde longitudinal 23 s'étend face aux pixels ouverts $31_i$, $31_{i+1}$ et face au pixel masqué $32_i$. Le guide d'onde longitudinal 23 s'étend selon l'axe longitudinal X. La dimension du guide d'onde longitudinal 23, selon l'axe Y, est inférieure ou égale à la dimension des pixels selon cet axe.
- un guide d'onde latéral 23' s'étend face aux pixels ouverts $31_k$, $31_{k+1}$ et face au pixel masqué $32_i$. Le guide d'onde latéral 23' s'étend selon l'axe latéral Y. La dimension du guide d'onde latéral 23', selon l'axe X, est inférieure ou égale à la dimension des pixels selon cet axe.

Face aux pixels ouverts $31_i$ et $31_{i+1}$ s'étend un premier réseau de diffraction $21_i$, $21_{i+1}$, apte à coupler l'onde lumineuse incidente $12_i$, $12_{i+1}$ dans le guide d'onde longitudinal 23. Ainsi, des ondes guidées $13'_i$, $13_{i+1}$ se propagent vers le pixel masqué $32_i$, comme décrit en lien avec le premier mode de réalisation.

Face aux pixels ouverts $31_k$ et $31_{k+1}$ s'étend un premier réseau de diffraction $21_k$, $21_{k+1}$, apte à coupler l'onde lumineuse incidente $12_k$, $12_{k+1}$ dans le guide d'onde latéral 23'. Ainsi, des ondes guidées $13'_k$, $13_{k+1}$ se propagent vers le pixel masqué $32_i$, de façon analogue à ce qui a été décrit en lien avec le premier mode de réalisation.

Face au pixel masqué $32_i$ s'étend un deuxième réseau de diffraction $22_i$ permettant de découpler l'onde se propageant dans le guide d'onde longitudinal 23 ainsi que l'onde se propageant dans le guide d'onde latéral 23'. Les ondes ainsi découplées se propagent vers le pixel masqué $32_i$.

Selon une configuration, les guides d'onde 23 et 23' sont coplanaires. Ils forment une grille comportant des bandes qui se croisent face à chaque pixel masqué $32_i$. Le deuxième réseau de diffraction $22_i$ est un même réseau bidimensionnel, disposé à l'intersection des guides d'onde 23 et 23'. La périodicité du réseau bidimensionnel est définie selon l'axe longitudinal X et l'axe latéral Y. Elle permet l'extraction des ondes guidées se propageant selon les deux axes, vers le pixel masqué $32_i$. De tels réseaux bidimensionnels, dans lesquels les inclusions prennent la forme de plots agencés de façon périodique selon l'axe X et l'axe Y, sont par exemple décrits dans le document EP3147646. Les réseaux de diffraction bidimensionnels sont connus de l'homme du métier.

Selon une configuration, notamment lorsque les guides d'onde 23 et 23' ne sont pas coplanaires. Le réseau de diffraction $22_i$ peut être constitué de deux réseaux de diffraction monodimensionnels, comme décrits en lien avec le premier mode de réalisation. Le réseau de diffraction $22_i$ comporte alors

- un réseau de diffraction monodimensionnel, permettant l'extraction des ondes guidées $13'_i$ et $13_{i+1}$ se propageant dans le guide d'onde longitudinal 23 ;
- un réseau de diffraction monodimensionnel, permettant l'extraction des ondes guidées $13'_k$ et $13_{k+1}$ se propageant dans le guide d'onde latéral 23'.

Selon un raisonnement analogue au mode de réalisation précédent :

- l'intensité détectée par le pixel $31_i$ est

$$I_i = (1 - 2\tau)^2 a_i^2 \; ; (10)$$

- l'intensité détectée par le pixel $31_{i+1}$ est

$$I_{i+1} = (1 - 2\tau)^2 a_{i+1}^2 \; ; (11)$$

- l'intensité détectée par le pixel $31_k$ est

$$I_k = (1 - 2\tau)^2 a_k^2 \; ; (12)$$

- l'intensité détectée par le pixel $31_{k+1}$ est

$$I_{k+1} = (1 - 2\tau)^2 a_{k+1}^2 \; (13)$$

- l'intensité détectée par le pixel $32_i$ est

$$I'_i = \tau^2 (a_i^2 + a_{i+1}^2 + a_k^2 + a_{k+1}^2 + 2a_i a_{i+1} \cos(\varphi_{i+1} - \varphi_i) + 2a_k a_{k+1} \cos(\varphi_{k+1} - \varphi_k) +$$
$$2a_i a_k \cos(\varphi_i - \varphi_k) + 2a_i a_{k+1} \cos(\varphi_i - \varphi_{k+1}) + 2a_{i+1} a_k \cos(\varphi_{i+1} - \varphi_k) +$$
$$2a_{i+1} a_{k+1} \cos(\varphi_{i+1} - \varphi_{k+1}) \, ) \, (14)$$

La figure 2B est une illustration d'un capteur d'image 20 dont les pixels sont agencés de la façon illustrée sur la figure 2A. On observe que le capteur 20 présente trois types de pixels :

- des pixels $31_\ell$ n'étant associés à aucun réseau de diffraction, ces derniers étant représentés en blanc, et permettant d'obtenir l'intensité $a_\ell^2$ de l'onde lumineuse qui leur est incidente.
- des pixels ouverts $31_i$, associés à un premier réseau de couplage, et dont l'intensité mesurée correspond à l'une des expressions (10) à (13).
- des pixels masqués $32_i$, de façon à obtenir une information relative à la phase, selon l'équation (14).

Un tel capteur permet d'obtenir une bonne résolution spatiale de l'intensité, le nombre de pixels permettant d'obtenir une information relative à l'intensité étant supérieur au nombre de pixels masqués. L'information de phase relative aux différents pixels masqués $32_i$ peut être combinée et former un système dont les inconnues sont les phases $\varphi_i$ des ondes lumineuses $12_i$ incidentes à chaque pixel ouvert $31_i$.

Sur la figure 2B, chaque flèche blanche représente la propagation d'une onde guidée entre un pixel ouvert et un pixel masqué qui lui est adjacent, selon une même ligne ou une même colonne.

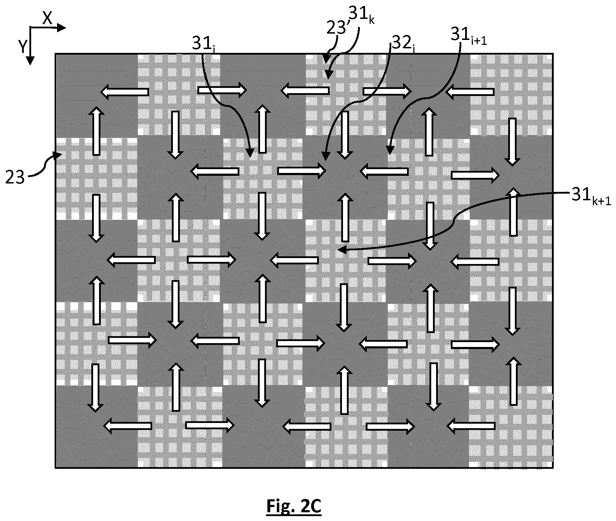

Selon une autre configuration, représentée sur la figure 2C, le nombre de pixels masqués $32_i$ est augmenté. A la différence de la configuration représentée sur les figures 2A et 2B, dans une telle configuration, chaque premier réseau de diffraction $21_i$ est un réseau bidimensionnel, permettant le couplage de l'onde lumineuse incidente $12_i$ dans un guide d'onde longitudinal 23 et dans un guide d'onde latéral 23'. De la même manière que sur la configuration précédente, les guides d'onde 23 et 23' s'étendent selon des bandes respectivement parallèlement à l'axe longitudinal X et l'axe latéral Y. La dimension de chaque guide d'onde perpendiculairement à l'axe selon lequel il s'étend, est inférieure ou égale à la dimension des pixels. Deux guides d'onde respectivement longitudinal et latéral se croisent face à chaque pixel ouvert $31_i$ et face à chaque pixel masqué $32_i$. A chaque pixel ouvert $31_i$ correspond un réseau de diffraction bidimensionnel $21_i$, permettant un couplage d'une partie de l'onde incidente $12_i$ dans un guide d'onde longitudinal 23 et dans un guide d'onde latéral 23'. A chaque pixel fermé $32_i$ correspond un réseau de diffraction bidimensionnel $22_i$, permettant une extraction des ondes guidées se propageant dans les guides d'onde 23, 23' et convergeant vers le réseau de diffraction bidimensionnel $22_i$. Il en résulte la formation d'une onde lumineuse découplée $14_i$ se propageant vers le pixel masqué $32_i$. La structure des guides d'onde bidimensionnels correspond à celle décrite en lien avec le deuxième guide d'onde $22_i$ représenté sur la figure 2A. Les intensités lumineuses détectées au niveau des pixels $31_i$, $31_{i+1}$, $31_k$, $31_{k+1}$ et $32_i$, représentés sur la figure 2C, correspondent aux expressions (10) à (14) explicitées dans le mode de réalisation précédent. Un tel mode de réalisation permet d'augmenter le nombre de pixels masqués, ce qui permet d'améliorer la résolution spatiale relative à l'information de phase.

Sur la figure 2C, chaque flèche blanche représente la propagation d'une onde guidée entre un pixel ouvert et un pixel masqué qui lui est adjacent, selon une même ligne ou une même colonne.

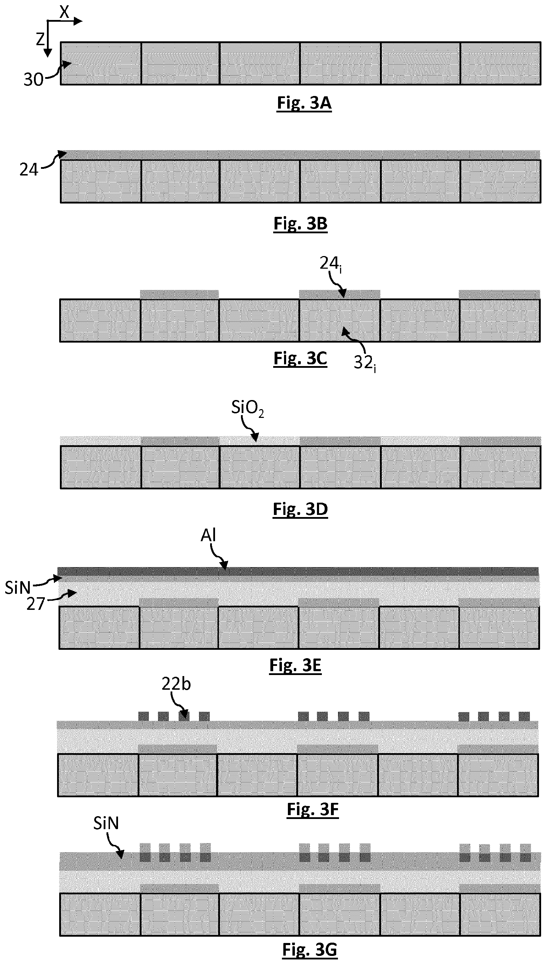

Les figures 3A à 3L schématisent les principales étapes d'un procédé de fabrication d'un capteur 20 tel que présenté dans les modes de réalisation qui précèdent.

Figure 3A : on part d'un substrat comportant une matrice 30 de photodiodes CMOS formant des pixels.

Figure 3B : on dépose une couche 24 de SiN, destinée à former la couche antireflet $24_i$ précédemment décrite. Cette étape est optionnelle.

Figure 3C : on grave la couche 24 de SiN, de façon à obtenir un plot antireflet $24_i$ au niveau de chaque pixel masqué $32_i$. De même que l'étape précédente, cette étape est optionnelle.

Figure 3D : dépôt d'une couche de $SiO_2$ entre chaque plot antireflet $24_i$ et polissage mécano-chimique (CMP Chemical-Mechanical Polish)

Figure 3E : dépôt d'une couche de $SiO_2$, formant la couche inférieure 27, puis dépôt de la couche de SiN, formant le guide d'onde 23, puis dépôt d'une couche d'Al, formant le deuxième matériau auxiliaire 22b du deuxième réseau de diffraction $22_i$.

Figure 3F : gravure de la couche d'Al, de façon à former le deuxième réseau de diffraction 22i face à chaque pixel masqué $32_i$.

Figure 3G : dépôt conforme d'une couche de SiN, formant le premier matériau auxiliaire 21b du premier réseau de diffraction $21_i$.

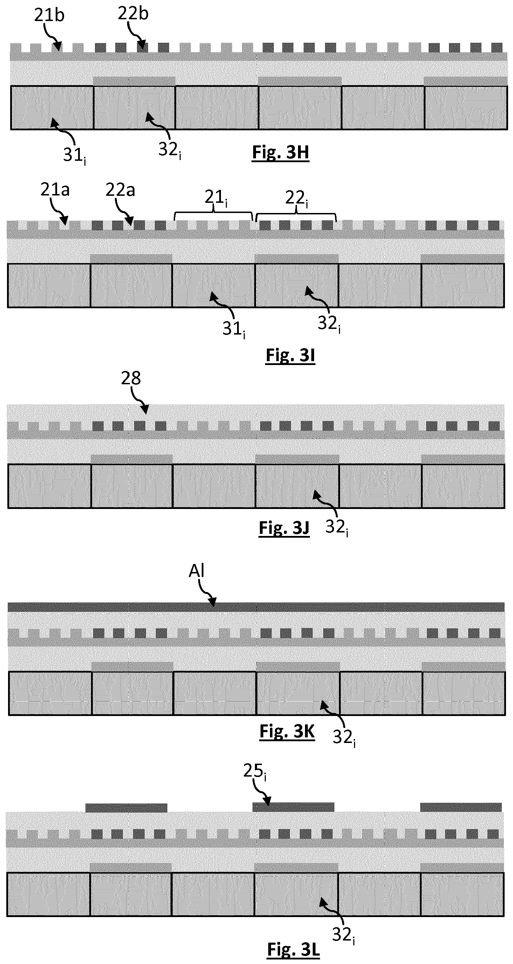

Figure 3H : gravure du SiN ;

Figure 3I : dépôt de $SiO_2$, formant le premier matériau 21a du premier réseau de diffraction $21_i$ et le deuxième matériau 22a du deuxième réseau de diffraction $22_i$, et polissage mécano-chimique.

Figure 3J : dépôt de $SiO_2$, pour former la couche supérieure 28.

Figure 3K : dépôt d'une couche d'aluminium, pour former le masque 25.

Figure 3L : gravure de la couche d'aluminium, de façon à former des plots $25_i$, chaque plot correspondant à un masque élémentaire $25_i$ s'étendant face à un pixel masqué $32_i$.

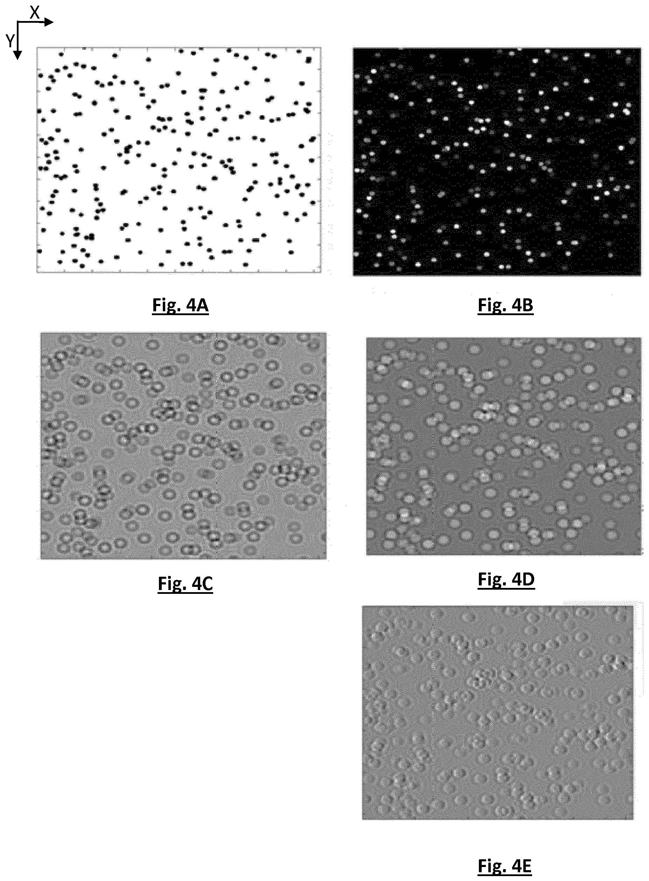

Les figures 4A à 4J illustrent des simulations d'images correspondant à l'observation d'un échantillon en mettant en œuvre le dispositif représenté sur la figure 1A. L'échantillon est modélisé par un ensemble de disques d'absorption constante et de phase aléatoire, répartis selon un plan, et de diamètre 10 $\mu$m. Les figures 4A et 4B représentent respectivement les distributions spatiales de l'absorption et de la phase. On a simulé le module et la phase d'un hologramme se formant selon le plan de détection P, ce qui correspond respectivement aux figures 4C et 4D. La figure 4C correspond à une image obtenue par les pixels ouverts (pixels d'intensité). La figure 4E représente une différence de phase entre deux pixels ouverts voisins d'une même ligne.

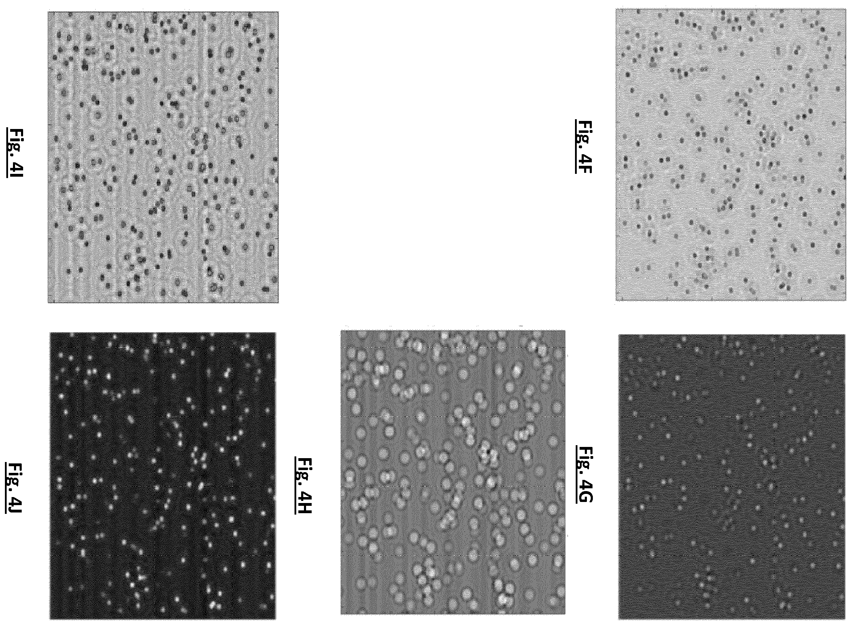

A partir du module de l'hologramme (figure 4C), on a appliqué un algorithme de reconstruction holographique, tel que décrit dans la demande de brevet WO2017162985. Cela permet d'obtenir une distribution spatiale du module et de la phase de l'onde lumineuse incidente au capteur d'image, dans le plan de l'échantillon. La distribution du module et de la phase correspond respectivement aux figures 4F et 4G.

L'image de la figure 4G permet d'obtenir une estimation de la phase de l'onde lumineuse, au niveau d'au moins un pixel par ligne du capteur d'image 20, cette estimation formant une phase de référence. Pour cela, il suffit de propager l'image de la figure 4G, dans le plan de détection, en certains pixels, de façon à obtenir la phase de référence. Connaissant au moins une phase de référence par ligne, et à partir des mesures de phases différentielles représentées sur la figure 4E, on obtient une distribution de la phase, au niveau de chaque pixel ouvert du capteur d'image. Cela est représenté sur la figure 4H.

A partir de la distribution du module de l'onde lumineuse incidente, dans le plan de détection, mesurée par le capteur (figure 4C) et de la distribution de la phase obtenue sur la figure 4H, on dispose d'une information de phase et de module au niveau du plan de détection. L'application d'un simple algorithme de propagation holographique permet de reconstruire une image du module (figure 4I) et une image de la phase (figure 4J) de l'onde lumineuse dans le plan de l'échantillon. On obtient alors une représentation exploitable de l'échantillon.

Ainsi, un point clef de l'invention est l'obtention, par des pixels masqués du capteur d'image, d'une intensité lumineuse représentative de la phase de l'onde lumineuse incidente au capteur. Plus précisément, l'intensité lumineuse mesurée correspond à une différence de phase entre des pixels adjacents de chaque pixel masqué. A partir d'une phase de référence, ou en résolvant un système d'équations, l'invention permet une estimation d'une distribution spatiale de la phase de l'onde lumineuse au niveau des pixels du capteur, tout en permettant également d'obtenir une distribution spatiale de l'intensité de l'onde lumineuse au niveau des pixels du capteur. Les informations relatives à la phase et à l'intensité, obtenues dans le plan de détection, permettent de propager l'onde lumineuse dans l'espace, et notamment dans le plan de l'échantillon, de façon à permettre une analyse de ce dernier.

Aussi, le capteur selon l'invention pourra s'appliquer dans l'observation d'échantillons, en particulier des échantillons transparents ou translucides. L'invention pourra être mise en œuvre dans le domaine de la biologie, par exemple dans l'observation de cellules ou de microorganismes, ou dans le domaine du diagnostic médical, en permettant une observation précise d'échantillons. Elle pourra également être mise en œuvre dans le contrôle de procédés industriels, ou dans le contrôle de l'environnement, dès lors que des échantillons transparents ou translucides sont analysés.

**Revendications**

1. Capteur d'image (20) comportant une matrice de pixels (30), s'étendant selon un plan de détection (P), et configurée pour former une image d'une onde lumineuse incidente (12), se propageant, dans une bande spectrale ($\Delta\lambda$), selon un axe de propagation (Z), le capteur d'image étant **caractérisé en ce qu'**il comporte un masque (25), formé de différents masques élémentaires opaques ($25_i$), s'étendant parallèlement au plan de détection, entre lesquels s'étendent des ouvertures ($26_i$), à travers lesquelles l'onde lumineuse incidente est apte à se propager en direction du plan de détection (P), la matrice de pixels étant divisée en :

   - des pixels ouverts ($31_i$) s'étendant face aux ouvertures ($26_i$);
   - des pixels masqués ($32_i$), chaque pixel masqué étant défini par une projection d'un masque élémentaire ($25_i$) selon l'axe de propagation (Z) sur la matrice de pixels, chaque pixel masqué étant associé au masque élémentaire lui faisant face ;

   le capteur d'image comportant, entre les pixels ouverts ($31_i$) et les ouvertures ($26_i$):

   - un guide d'onde (23), formant une bande s'étendant face à des pixels masqués et à des pixels ouverts ;
   - un premier réseau de diffraction ($21_i$), s'étendant face à au moins un pixel ($31_i$) ouvert, et configuré pour coupler une partie de l'onde lumineuse incidente ($12_i$) dans le guide d'onde (23), de manière à former une onde guidée ($13'_i$, $13_{i+1}$), le premier réseau de diffraction étant configuré pour transmettre une autre partie de l'onde lumineuse incidente ($12'_i$) vers le pixel ($31_i$) ouvert, le premier réseau de diffraction étant associé au pixel ouvert ($31_i$) ;
   - un deuxième réseau de diffraction ($22_i$), s'étendant face à un pixel masqué ($32_i$), et configuré pour extraire une partie de l'onde guidée ($13'_i$, $13_{i+1}$), se propageant dans le guide d'onde (23), de telle sorte que l'onde ainsi extraite ($14_i$) se propage vers le pixel masqué ($32_i$), le deuxième réseau de diffraction étant associé au pixel masqué ($32_i$).

2. Capteur d'image selon la revendication 1, dans lequel :

   - un pixel masqué ($32_i$) s'étend entre deux pixels ouverts ($31_i$, $31_{i+1}$), qui lui sont adjacents, chaque pixel ouvert étant associé à un premier réseau de diffraction ($21_i$, $21_{i+1}$);
   - le guide d'onde (23) s'étend face aux deux pixels ouverts et face au pixel masqué ($32_i$);
   - le pixel masqué ($32_i$) est associé à un deuxième réseau de diffraction ($22_i$), de façon à extraire des ondes lumineuses ($13'_i$, $13_{i+1}$) guidées dans le guide d'onde, résultant respectivement d'un couplage de l'onde lumineuse incidente par le premier réseau de diffraction associé à chaque pixel ouvert ($31_i$, $31_{i+1}$) adjacent du pixel masqué.

3. Capteur d'image selon la revendication 2 dans lequel le pixel masqué ($32_i$) et les pixels ouverts ($31_i$, $31_{i+1}$) lui étant adjacents sont disposés selon une même ligne ou selon une même colonne de la matrice de pixels (30).

4. Capteur d'image selon l'une quelconque des revendications précédentes, dans lequel :

   - un pixel masqué ($32_i$) s'étend entre deux pixels ouverts ($31_i$, $31_{i+1}$), qui lui sont adjacents, selon une ligne de la matrice de pixels, chaque pixel ouvert étant associé à un premier réseau de diffraction ($21_i$, $21_{i+1}$);
   - le guide d'onde (23) s'étend face aux deux pixels ouverts et face au pixel masqué ($32_i$), parallèlement à la ligne, formant un guide d'onde longitudinal, chaque premier réseau de diffraction ($21_i$, $21_{i+1}$) associé aux pixels ouverts de la ligne étant configuré pour coupler une partie de l'onde lumineuse incidente ($12_i$) dans le guide d'onde longitudinal (23) ;
   - le pixel masqué ($32_i$) est associé à un deuxième réseau de diffraction ($22_i$), de façon à extraire des ondes lumineuses guidées ($13'_i$, $13_{i+1}$) dans le guide d'onde longitudinal (23), de telle sorte que les ondes lumineuses ainsi extraites soient détectées par le pixel masqué (32$_i$);
- le pixel masqué s'étend entre deux pixels ouverts (31$_k$, 31$_{k+1}$) qui lui sont adjacents, selon une colonne de la matrice de pixels, lesdits pixels ouverts de la colonne étant associés à un premier réseau de diffraction (21$_k$, 21$_{k+1}$) ;
- le capteur comporte un guide d'onde (23'), dit guide d'onde latéral, s'étendant parallèlement à la colonne, face aux deux pixels ouverts de la colonne et face au pixel masqué (32$_i$), chaque premier réseau de diffraction associé aux pixels ouverts de la colonne étant configuré pour coupler une partie de l'onde lumineuse incidente (12$_i$) dans le guide d'onde latéral (23') ;
- le deuxième réseau de diffraction (22$_i$) est configuré pour extraire des ondes lumineuses (13'$_k$, 13$_{k+1}$) se propageant dans le guide d'onde latéral (23'), de telle sorte que les ondes lumineuses ainsi extraites soient détectées par le pixel masqué (32$_i$).

5. Capteur d'image selon l'une quelconque des revendications précédentes, dans lequel :

- le premier réseau de diffraction (21$_i$) est formé par une première couche mince, s'étendant parallèlement au guide d'onde (23), et formée d'un premier matériau (21a), la première couche mince comportant des inclusions d'un premier matériau auxiliaire (21b), les indices de réfraction respectifs du premier matériau et du premier matériau auxiliaire étant différents, le premier matériau et le premier matériau auxiliaire étant transparents dans tout ou partie de la bande spectrale (Δλ) ;
- le deuxième réseau de diffraction (22$_i$) est formé par une deuxième couche mince, s'étendant parallèlement au guide d'onde (23), formée d'un deuxième matériau (22a), la deuxième couche mince comportant des inclusions selon un deuxième matériau auxiliaire (22b), les indices de réfraction respectifs du deuxième matériau et du deuxième matériau auxiliaire étant différents.

6. Capteur d'image selon la revendication 5, dans lequel le premier matériau (21a) et le deuxième matériau (22a) forment un même matériau.

7. Capteur d'image selon l'une quelconque des revendications 5 ou 6, dans lequel le deuxième matériau auxiliaire (22b) est un métal.

8. Capteur d'image selon l'une quelconque des revendications 1 à 7, dans lequel le premier réseau de diffraction et le deuxième réseau de diffraction s'étendent selon un même plan.

9. Capteur d'image selon l'une quelconque des revendications 1 à 8, dans lequel le premier réseau de diffraction et le deuxième réseau de diffraction s'étendent parallèlement à la matrice de pixels.

10. Capteur d'image selon l'une quelconque des revendications précédentes, dans lequel au moins un masque élémentaire (25$_i$) comporte une face réfléchissante, faisant face à un pixel masqué (32$_i$) associé au masque élémentaire, de façon à renvoyer une partie d'onde lumineuse, extraite du deuxième guide d'onde (22$_i$) s'étendant face au pixel masqué, vers ce dernier.

11. Dispositif d'observation d'un échantillon (5), comportant :

- une source de lumière, configurée pour émettre une onde lumineuse (11), dite onde lumineuse d'illumination, selon une bande spectrale (Δλ), selon un axe de propagation (Z), vers l'échantillon (5) ;
- un capteur d'image (20);
- un support (5s), apte à recevoir l'échantillon (5), de telle sorte que l'échantillon s'étend entre la source de lumière (10) et l'échantillon (5), le capteur d'image étant configuré pour détecter une onde lumineuse incidente (12$_i$) se propageant entre l'échantillon (5) et le capteur (20) lorsque l'échantillon (5) est illuminé par l'onde lumineuse d'illumination (11) ;
le capteur d'image étant un capteur selon l'une quelconque des revendications 1 à 10.

12. Procédé de détermination d'une intensité et d'une phase d'une onde lumineuse (12$_i$, 12$_{i+1}$), en utilisant un capteur d'image (20) selon l'une quelconque des revendications 1 à 10, le capteur d'image étant tel que :

- un pixel masqué (32$_i$) s'étend, selon une ligne ou une colonne, entre deux pixels ouverts (31$_i$, 31$_{i+1}$);
- le guide d'onde (23) s'étend, face aux pixels ouverts (31$_i$, 31$_{i+1}$) et face au pixel masqué (32$_i$), selon ladite ligne ou colonne ;

- les pixels ouverts ($31_i$, $31_{i+1}$) sont chacun associés à un premier réseau de diffraction ($21_i$, $21_{i+1}$);
- le pixel masqué ($32_i$) est associé à un deuxième réseau de diffraction ($22_i$);

le procédé comportant les étapes suivantes :

a) illumination du capteur (20) de telle sorte qu'une onde lumineuse incidente ($12_i$, $12_{i+1}$) se propage vers chaque pixel ouvert ($31_i$, $31_{i+1}$);

b) couplage d'une partie de l'onde lumineuse incidente ($12_i$, $12_{i+1}$) le premier réseau de diffraction ($21_i$, $21_{i+1}$) s'étendant face à chaque pixel ouvert, de façon à former, au niveau du premier réseau de diffraction ($21_i$, $21_{i+1}$), une onde lumineuse confinée ($13'_i$, $13_{i+1},$) se propageant dans le guide d'onde (23), et à transmettre, au pixel ouvert associé au premier réseau de diffraction, une onde lumineuse dite transmise ($12'_i$, $12'_{i+1}$);

c) extraction, par le deuxième réseau de diffraction, d'une partie de chaque onde lumineuse confinée ($13'_i$, $13_{i+1}$), se propageant dans le guide d'onde (23), de façon à former une onde lumineuse extraite ($14_i$) ;

d) détection de l'onde lumineuse extraite ($14_i$) par le pixel masqué ($32_i$);

e) à partir d'une intensité de ($I'_i$) de l'onde lumineuse extraite ($14_i$), obtention d'une information relative à différence de phase ($\varphi_{i+1}$-$\varphi_i$) de l'onde lumineuse incidente ($12_i$, $12_{i+1}$) se propageant respectivement vers chaque pixel ouvert ($31_i$, $31_{i+1}$) ;

f) détection l'onde lumineuse transmise ($12'_i$, $12'_{i+1}$) par le premier réseau de diffraction, vers chaque pixel ouvert ($31_i$, $31_{i+1}$) associé au premier réseau de diffraction, de façon à obtenir une intensité de l'onde lumineuse transmise ($I_i$, $I_{i+1}$), représentative de l'intensité de l'onde lumineuse incidente ($12_i$, $12_{i+1}$) au pixel ouvert.

13. Procédé selon la revendication 12, comportant une étape :
g) à partir de la différence de phases ($\varphi_{i+1}$-$\varphi_i$,) obtenue lors de l'étape e), estimation d'une phase de l'onde lumineuse détectée par chaque pixel ouvert.

14. Procédé selon la revendication 13, dans lequel dans lequel les pixels ouverts ($31_i$, $31_{i+1}$) sont disposés selon une même ligne ou une même colonne de la matrice de pixels (30), l'étape g) comportant une prise en compte d'une valeur de phase, dite de référence ($\varphi_{ref}$), obtenue sur un pixel de la ligne ou de la colonne.

15. Procédé selon l'une quelconque des revendications 13 ou 14, dans lequel le capteur comporte différents pixels masqués ($32_i$), chaque pixel masqué étant disposé entre au moins deux pixels ouverts ($31_i$, $31_{i+1}$) selon une même ligne et deux pixels ouverts ($31_k$, $31_{k+1}$) selon une même colonne, les étapes a) à f) étant mises en œuvre sur chaque pixel masqué ($32_i$) ainsi que sur les pixels ouverts ($31_i$, $31_{i+1}$, $31_k$, $31_{k+1}$) entre lesquels le pixel masqué est disposé, l'étape g) étant réalisée à partir d'une différence de phase déterminée à partir d'une intensité ($I'_i$) d'une onde lumineuse détectée par chaque pixel masqué.

## Patentansprüche

1. Bilderfassungssensor (20), der eine Pixelmatrix (30) aufweist, die sich entlang einer Erkennungsebene (P) erstreckt, und ausgebildet ist, um ein Bild einer einfallenden Lichtwelle (12) zu bilden, die sich in einem Spektralband ($\Delta\lambda$) entlang einer Ausbreitungsachse (Z) ausbreitet, wobei der Bilderfassungssensor **dadurch gekennzeichnet ist, dass** er eine Maske (25) aufweist, die aus verschiedenen lichtundurchlässigen Elementarmasken ($25_i$) gebildet wird, die sich parallel zur Erkennungsebene erstrecken und zwischen denen sich Öffnungen ($26_i$) erstrecken, durch die die einfallende Lichtwelle in die Lage versetzt wird, sich in Richtung der Erkennungsebene (P) auszubreiten, wobei die Pixelmatrix unterteilt ist in:

- offene Pixel ($31_i$), die sich gegenüber den Öffnungen ($26_i$) erstrecken;
- maskierte Pixel ($32_i$), wobei jedes maskierte Pixel durch eine Projektion einer Elementarmaske ($25_i$) entlang der Ausbreitungsachse (Z) auf der Pixelmatrix definiert ist, wobei jedes maskierte Pixel der ihm gegenüberliegenden Elementarmaske zugeordnet ist;

wobei der Bilderfassungssensor zwischen den offenen Pixeln ($31_i$) und den Öffnungen ($26_i$) Folgendes aufweist:

- einen Wellenleiter (23), der ein Band bildet, das sich gegenüber maskierten Pixeln und offenen Pixeln erstreckt;
- ein erstes Beugungsgitter ($21_i$), das sich gegenüber von mindestens einem offenen Pixel ($31_i$) erstreckt und ausgebildet ist, um einen Teil der einfallenden Lichtwelle ($12_i$) so in den Wellenleiter (23) einzukoppeln, dass eine geführte Welle ($13'_i$, $13_{i+1}$) gebildet wird, wobei das erste Beugungsgitter ausgebildet ist, um einen anderen Teil der einfallenden Lichtwelle (12'$_i$) zu dem offenen Pixel (31$_i$) zu übertragen, wobei das erste Beugungsgitter dem offenen Pixel (31$_i$) zugeordnet ist;
- ein zweites Beugungsgitter (22$_i$), das sich gegenüber einem maskierten Pixel (32$_i$) erstreckt und ausgebildet ist, um einen Teil der geführten Welle (13'$_i$, 13$_{i+1}$) zu extrahieren, die sich in dem Wellenleiter (23) ausbreitet, sodass sich die so extrahierte Welle (14$_i$) zu dem maskierten Pixel (32$_i$) hin ausbreitet, wobei das zweite Beugungsgitter dem maskierten Pixel (32$_i$) zugeordnet ist.

2. Bilderfassungssensor nach Anspruch 1, wobei:

- sich ein maskiertes Pixel (32i) zwischen zwei benachbarten offenen Pixeln (31$_i$, 31$_{i+1}$) erstreckt, wobei jedes offene Pixel einem ersten Beugungsgitter (21$_i$, 21$_{i+1}$) zugeordnet ist;
- sich der Wellenleiter (23) gegenüber den beiden offenen Pixeln und gegenüber dem maskierten Pixel (32$_i$) erstreckt;
- das maskierte Pixel (32$_i$) einem zweiten Beugungsgitter (22$_i$) zugeordnet ist, um im Wellenleiter geführte Lichtwellen (13'$_i$, 13$_{i+1}$) zu extrahieren, die jeweils aus einer Kopplung der durch das erste Beugungsgitter einfallenden Lichtwelle resultieren, das jedem offenen Pixel (31$_i$, 31$_{i+1}$) neben dem maskierten Pixel zugeordnet ist.

3. Bilderfassungssensor nach Anspruch 2, wobei das maskierte Pixel (32$_i$) und die benachbarten offenen Pixel (31$_i$, 31$_{i+1}$) in derselben Zeile oder in derselben Spalte der Pixelmatrix (30) angeordnet sind.

4. Bilderfassungssensor nach einem der vorhergehenden Ansprüche, wobei:

- sich ein maskiertes Pixel (32$_i$) zwischen zwei benachbarten offenen Pixeln (31$_i$, 31$_{i+1}$) in einer Zeile der Pixelmatrix erstreckt, wobei jedes offene Pixel einem ersten Beugungsgitter (21$_i$, 21$_{i+1}$) zugeordnet ist;
- sich der Wellenleiter (23) gegenüber den beiden offenen Pixeln und gegenüber dem maskierten Pixel (32$_i$) parallel zu der Zeile erstreckt und einen Longitudinalwellenleiter bildet, wobei jedes erste Beugungsgitter (21$_i$, 21$_{i+1}$), das den offenen Pixeln der Zeile zugeordnet ist, ausgebildet ist, um einen Teil der einfallenden Lichtwelle (12$_i$) in den Longitudinalwellenleiter (23) einzukoppeln;
- das maskierte Pixel (32$_i$) einem zweiten Beugungsgitter (22$_i$) zugeordnet ist, um im Longitudinalwellenleiter (23) geführte Lichtwellen (13'$_i$, 13$_{i+1}$) zu extrahieren, sodass die so extrahierten Lichtwellen von dem maskierten Pixel (32i) erkannt werden;
- sich das maskierte Pixel zwischen zwei benachbarten offenen Pixeln (31$_k$, 31$_{k+1}$) in einer Spalte der Pixelmatrix erstreckt, wobei die offenen Pixel der Spalte einem ersten Beugungsgitter (21$_k$, 21$_{k+1}$) zugeordnet sind;
- der Sensor einen Wellenleiter (23') aufweist, der als Lateralwellenleiter bezeichnet wird und sich parallel zu der Spalte gegenüber den beiden offenen Pixeln der Spalte und gegenüber dem maskierten Pixel (32$_i$) erstreckt, wobei jedes erste Beugungsgitter, das den offenen Pixeln der Spalte zugeordnet ist, ausgebildet ist, um einen Teil der einfallenden Lichtwelle (12$_i$) in den Lateralwellenleiter (23') einzukoppeln;
- das zweite Beugungsgitter (22$_i$) ausgebildet ist, um sich im Lateralwellenleiter (23') ausbreitende Lichtwellen (13'$_k$, 13$_{k+1}$) zu extrahieren, sodass die so extrahierten Lichtwellen von dem maskierten Pixel (32$_i$) erkannt werden.

5. Bilderfassungssensor nach einem der vorhergehenden Ansprüche, wobei:

- das erste Beugungsgitter (21$_i$) durch eine erste dünne Schicht gebildet wird, die sich parallel zum Wellenleiter (23) erstreckt und durch ein erstes Material (21a) gebildet wird, wobei die erste dünne Schicht Einschlüsse eines ersten Hilfsmaterials (21b) aufweist, wobei die jeweiligen Brechungsindizes des ersten Materials und des ersten Hilfsmaterials unterschiedlich sind, wobei das erste Material und das erste Hilfsmaterial im gesamten oder einem Teil des Spektralbandes ($\Delta\lambda$) transparent sind;
- das zweite Beugungsgitter (22$_i$) durch eine zweite dünne Schicht gebildet wird, die sich parallel zum Wellenleiter (23) erstreckt und durch ein zweites Material (22a) gebildet wird, wobei die zweite dünne Schicht Einschlüsse eines zweiten Hilfsmaterials (22b) aufweist, wobei die jeweiligen Brechungsindizes des zweiten Materials und des zweiten Hilfsmaterials unterschiedlich sind.

6. Bilderfassungssensor nach Anspruch 5, wobei das erste Material (21a) und das zweites Material (22a) dasselbe Material bilden.

7. Bilderfassungssensor nach einem der Ansprüche 5 oder 6, wobei das zweite Hilfsmaterial (22b) ein Metall ist.

8. Bilderfassungssensor nach einem der Ansprüche 1 bis 7, wobei sich das erste Beugungsgitter und das zweite Beugungsgitter in derselben Ebene erstrecken.

9. Bilderfassungssensor nach einem der Ansprüche 1 bis 8, wobei sich das erste Beugungsgitter und das zweite Beugungsgitter parallel zur Pixelmatrix erstrecken.

10. Bilderfassungssensor nach einem der vorhergehenden Ansprüche, wobei mindestens eine Elementarmaske ($25_i$) eine reflektierende Fläche aufweist, die einem maskierten Pixel ($32_i$) gegenüberliegt, das der Elementarmaske zugeordnet ist, um einen Teil der Lichtwelle, die aus dem zweiten Lichtleiter ($22_i$) extrahiert wird, der sich gegenüber dem maskierten Pixel erstreckt, zu diesem zurückzugeben.

11. Vorrichtung zum Beobachten einer Probe (5), die Folgendes aufweist:

- eine Lichtquelle, ausgebildet zum Aussenden einer Lichtwelle (11), die als Beleuchtungslichtwelle bezeichnet wird, in einem Spektralband ($\Delta\lambda$) entlang einer Ausbreitungsachse (Z) zu einer Probe (5);
- einen Bilderfassungssensor (20);
- einen Träger (5s), der geeignet ist, die Probe (5) so aufzunehmen, dass sich die Probe zwischen der Lichtquelle (10) der Probe (5) erstreckt, wobei der Bilderfassungssensor ausgebildet ist, eine einfallende Lichtwelle ($12_i$) zu erkennen, die sich zwischen der Probe (5) und dem Sensor (20) ausbreitet, wenn die Probe (5) von der Beleuchtungslichtquelle (11) beleuchtet wird;

wobei der Bilderfassungssensor ein Sensor nach einem der Ansprüche 1 bis 10 ist.

12. Verfahren zum Bestimmen einer Intensität und einer Phase einer Lichtwelle ($12_i$, $12_{i+1}$), unter Verwendung eines Bilderfassungssensors (20) gemäß einem der Ansprüche 1 bis 10, wobei der Bilderfassungssensor so aufgebaut ist, dass:

- sich ein maskiertes Pixel ($32_i$) in einer Zeile oder Spalte zwischen zwei offenen Pixeln ($31_i$, $31_{i+1}$) erstreckt;
- sich der Wellenleiter (23) in dieser Zeile oder Spalte gegenüber den offenen Pixeln ($31_i$, $31_{i+1}$) und gegenüber dem maskierten Pixel ($32_i$) erstreckt;
- die offenen Pixel ($31_i$, $31_{i+1}$) jeweils einem ersten Beugungsgitter ($21_i$, $21_{i+1}$) zugeordnet sind;
- das maskierte Pixel ($32i$) einem zweiten Beugungsgitter ($22i$) zugeordnet ist;

das Verfahren die folgenden Schritte aufweist:

a) Beleuchten des Sensors (20) in der Art, dass sich eine einfallende Lichtwelle ($12_i$, $12_{i+1}$) zu jedem offenen Pixel ($31_i$, $31_{i+1}$) hin ausbreitet;
b) Koppeln eines Teils der einfallenden Lichtwelle ($12_i$, $12_{i+1}$), wobei sich das erste Beugungsgitter ($21_i$, $21_{i+1}$) gegenüber jedem offenen Pixel erstreckt, um auf der Ebene des ersten Beugungsgitters ($21_i$, $21_{i+1}$) eine begrenzte Lichtwelle ($13'_i$, $13_{i+1}$) zu bilden, die sich in dem Wellenleiter (23) ausbreitet, und um an das offene Pixel, das dem ersten Beugungsgitter zugeordnet ist, eine Lichtwelle zu übertragen, die als übertragene Lichtwelle ($12'_i$, $12'_{i+1}$) bezeichnet wird;
c) Extrahieren eines Teils von jeder begrenzten Lichtwelle ($13'_i$, $13_{i+1}$), die sich in dem Wellenleiter (23) ausbreitet, durch das zweite Beugungsgitter, um eine extrahierte Lichtwelle ($14_i$) zu bilden;
d) Erkennen der extrahierten Lichtwelle ($14_i$) durch das maskierte Pixel ($32_i$);
e) aus einer Intensität von ($I'_i$) der extrahierten Lichtwelle ($14_i$) Erhalten einer Information bezüglich der Phasendifferenz ($\phi_{i+1}-\phi_i$) der einfallenden Lichtwelle ($12_i$, $12_{i+1}$), die sich jeweils zu jedem offenen Pixel ($31_i$, $31_{i+1}$) hin ausbreitet;
f) Erkennen der durch das erste Beugungsgitter zu jedem offenen Pixel ($31_i$, $31_{i+1}$), das dem ersten Beugungsgitter zugeordnet ist, übertragenen Lichtwelle ($12'_i$, $12'_{i+1}$), um eine Intensität der übertragenen Lichtwelle ($I_i$, $I_{i+1}$) zu erhalten, die die Intensität der am offenen Pixel einfallenden Lichtwelle ($12_i$, $12_{i+1}$) darstellt.

13. Verfahren nach Anspruch 12, das folgenden Schritt aufweist:
g) ausgehend von der Phasendifferenz ($\phi_{i+1}-\phi_i$), die in Schritt e) erhalten wird, Abschätzen einer Phase der Lichtwelle, die von jedem offenen Pixel erkannt wird.

14. Verfahren nach Anspruch 13, wobei die offenen Pixel ($31_i$, $31_{i+1}$) in derselben Zeile oder in derselben Spalte der Pixelmatrix (30) angeordnet sind und Schritt g) eine Berücksichtigung eines Phasenwerts aufweist, der als Referenz

(ϕ*ref*) bezeichnet wird und auf einem Pixel der Zeile oder der Spalte erhalten wird.

**15.** Verfahren nach einem der Ansprüche 13 oder 14, wobei der Sensor verschiedene maskierte Pixel ($32_i$) aufweist, wobei jedes maskierte Pixel zwischen mindestens zwei offenen Pixeln ($31_i$, $31_{i+1}$) in derselben Zeile und zwei offenen Pixeln ($31_k$, $31_{k+1}$) in derselben Spalte angeordnet ist, wobei die Schritte a) bis f) auf jedes maskierte Pixel ($32_i$) und auf die offenen Pixel ($31_i$, $31_{i+1}$, $31_k$, $31_{k+1}$) angewendet wird, zwischen denen das maskierte Pixel angeordnet ist, wobei Schritt g) ausgehend von einer Phasendifferenz durchgeführt wird, die ausgehend von einer Intensität ($I'_i$) einer Lichtwelle bestimmt wird, die von jedem maskierten Pixel erkannt wird.

## Claims

**1.** Image sensor (20) comprising a matrix of pixels (30), extending along a detection plane (P), and configured to form an image of an incident light wave (12) propagating, in a spectral band ($\Delta\lambda$), along a propagation axis (Z), the image sensor being **characterized in that** it comprises a mask (25), formed by different opaque elementary masks ($25_i$), extending parallel to the detection plane, between which masks openings ($26_i$) extend, through which openings the incident light wave can propagate toward the detection plane (P), the matrix of pixels being divided into:

- open pixels ($31_i$) extending facing the openings ($26_i$);
- masked pixels ($32_i$), each masked pixel being defined by a projection of an elementary mask ($25_i$) along the propagation axis (Z) on the matrix of pixels, each masked pixel being associated with the elementary mask facing it;

the image sensor comprising, between the open pixels ($31_i$) and the openings ($26_i$):

- a waveguide (23), forming a strip extending facing masked pixels and open pixels;
- a first diffraction grating ($21_i$), extending facing at least one open pixel ($31_i$), and configured to couple part of the incident light wave ($12_i$) into the waveguide (23) so as to form a guided wave ($13'_i$, $13_{i+1}$), the first diffraction grating being configured to transmit another part of the incident light wave ($12'_i$) toward the open pixel ($31_i$), the first diffraction grating being associated with the open pixel ($31_i$);
- a second diffraction grating ($22_i$), extending facing a masked pixel ($32_i$), and configured to extract part of the guided wave ($13'_i$, $13_{i+1}$) propagating in the waveguide (23), so that the wave ($14_i$) extracted in this way propagates toward the masked pixel ($32_i$), the second diffraction grating being associated with the masked pixel ($32_i$).

**2.** Image sensor according to Claim 1, wherein:

- a masked pixel ($32_i$) extends between two open pixels ($31_i$, $31_{i+1}$) which are adjacent to it, each open pixel being associated with a first diffraction grating ($21_i$, $21_{i+1}$);
- the waveguide (23) extends facing the two open pixels and facing the masked pixel (32i);
- the masked pixel ($32_i$) is associated with a second diffraction grating ($22_i$), so as to extract light waves ($13'_i$, $13_{i+1}$) guided in the waveguide, resulting, respectively, from a coupling of the incident light wave by the first diffraction grating associated with each open pixel ($31_i$, $31_{i+1}$) adjacent to the masked pixel.

**3.** Image sensor according to Claim 2, wherein the masked pixel ($32_i$) and the open pixels ($31_i$, $31_{i+1}$) adjacent to it are arranged along the same row or along the same column of the matrix of pixels (30).

**4.** Image sensor according to any one of the preceding claims, wherein:

- a masked pixel ($32_i$) extends between two open pixels ($31_i$, $31_{i+1}$), which are adjacent to it, along a row of the matrix of pixels, each open pixel being associated with a first diffraction grating ($21_i$, $21_{i+1}$);
- the waveguide (23) extends facing the two open pixels and facing the masked pixel ($32_i$), parallel to the row, forming a longitudinal waveguide, each first diffraction grating ($21_i$, $21_{i+1}$) associated with the open pixels of the row being configured to couple part of the incident light wave ($12_i$) into the longitudinal waveguide (23);
- the masked pixel ($32_i$) is associated with a second diffraction grating ($22_i$), so as to extract light waves ($13'_i$, $13_{i+1}$) guided in the longitudinal waveguide (23), in such a way that the light waves extracted in this way are detected by the masked pixel ($32_i$);
- the masked pixel extends between two open pixels ($31_k$, $31_{k+1}$), which are adjacent to it, along a column of the matrix of pixels, said open pixels of the column being associated with a first diffraction grating ($21_k$, $21_{k+1}$);

- the sensor comprises a waveguide (23'), called lateral waveguide, extending parallel to the column, facing the two open pixels of the column and facing the masked pixel (32$_i$), each first diffraction grating associated with the open pixels of the column being configured to couple part of the incident light wave (12$_i$) into the lateral waveguide (23');
- the second diffraction grating (22$_i$) is configured to extract light waves (13'$_k$, 13$_{k+1}$) propagating in the lateral waveguide (23'), in such a way that the light waves extracted in this way are detected by the masked pixel (32$_i$).

5. Image sensor according to any one of the preceding claims, wherein:

- the first diffraction grating (21$_i$) is formed by a first thin layer, extending parallel to the waveguide (23), and formed from a first material (21a), the first thin layer comprising inclusions of a first auxiliary material (21b), the respective refractive indices of the first material and of the first auxiliary material being different, the first material and the first auxiliary material being transparent in part or all of the spectral band ($\Delta\lambda$);
- the second diffraction grating (22$_i$) is formed by a second thin layer, extending parallel to the waveguide (23), formed from a second material (22a), the second thin layer comprising inclusions of a second auxiliary material (22b), the respective refractive indices of the second material and of the second auxiliary material being different.

6. Image sensor according to Claim 5, wherein the first material (21a) and the second material (22a) form the same material.

7. Image sensor according to either one of Claims 5 and 6, wherein the second auxiliary material (22b) is a metal.

8. Image sensor according to any one of Claims 1 to 7, wherein the first diffraction grating and the second diffraction grating extend along the same plane.

9. Image sensor according to any one of Claims 1 to 8, wherein the first diffraction grating and the second diffraction grating extend parallel to the matrix of pixels.

10. Image sensor according to any one of the preceding claims, wherein at least one elementary mask (25$_i$) comprises a reflective face, facing a masked pixel (32$_i$) associated with the elementary mask, so as to reflect part of the light wave extracted from the second waveguide (22$_i$), extending facing the masked pixel, toward the latter.

11. Device for observing a sample (5), comprising:

- a light source configured to emit a light wave (11), called illuminating light wave, in a spectral band ($\Delta\lambda$), along a propagation axis (Z), toward the sample (5);
- an image sensor (20);
- a holder (5s) able to receive the sample (5), so that the sample extends between the light source (10) and the sample (5), the image sensor being configured to detect an incident light wave (12$_i$) propagating between the sample (5) and the sensor (20) when the sample (5) is illuminated by the illuminating light wave (11); the image sensor being a sensor according to any one of Claims 1 to 10.

12. Method for determining an intensity and a phase of a light wave (12$_i$, 12$_{i+1}$), using an image sensor (20) according to any one of Claims 1 to 10, the image sensor being such that:

- a masked pixel (32$_i$) extends along a row or a column between two open pixels (31$_i$, 31$_{i+1}$);
- the waveguide (23) extends, facing the open pixels (31$_i$, 31$_{i+1}$) and facing the masked pixel (32$_i$), along said row or column;
- each of the open pixels (31$_i$, 31$_{i+1}$) are associated with a first diffraction grating (21$_i$, 21$_{i+1}$);
- the masked pixel (32$_i$) is associated with a second diffraction grating (22$_i$);

the method comprising the following steps:

a) illuminating the sensor (20) in such a way that an incident light wave (12$_i$, 12$_{i+1}$) propagates towards each open pixel (31$_i$, 31$_{i+1}$);
b) coupling part of the incident light wave (12$_i$, 12$_{i+1}$), the first diffraction grating (21$_i$, 21$_{i+1}$) extending facing each open pixel, in such a way as to form, at the first diffraction grating (21$_i$, 21$_{i+1}$), a confined light wave (13'$_i$, 13$_{i+1}$) propagating in the waveguide (23) and to transmit what is called a transmitted light wave (12'$_i$, 12'$_{i+1}$) to

the open pixel associated with the first diffraction grating;

c) part of each confined light wave ($13'_i$, $13_{i+1}$) propagating in the waveguide (23) is extracted by means of the second diffraction grating, so as to form an extracted light wave ($14_i$);

d) the extracted light wave ($14_i$) is detected by the masked pixel ($32_i$);

e) on the basis of an intensity ($I'i$) of the extracted light wave ($14_i$), information is obtained relating to the phase difference ($\varphi_{i+1}$-$\varphi_i$) of the incident light wave ($12_i$, $12_{i+1}$) propagating towards each open pixel ($31_i$, $31_{i+1}$), respectively;

f) the light wave ($12'_i$, $12'_{i+1}$) transmitted by the first diffraction grating towards each open pixel ($31_i$, $31_{i+1}$) associated with the first diffraction grating is detected, in order to obtain an intensity of the transmitted light wave ($I_i$, $I_{i+1}$) representative of the intensity of the light wave ($12_i$, $12_{i+1}$) incident on the open pixel.

13. Method according to Claim 12, comprising a step of:

g) estimating a phase of the light wave detected by each open pixel on the basis of the phase difference ($\varphi_{i+1}$-$\varphi_i$) obtained in step e).

14. Method according to Claim 13, wherein the open pixels ($31_i$, $31_{i+1}$) are positioned along the same row or the same column of the matrix of pixels (30), step g) comprising taking into account a phase value, called reference phase value ($\varphi_{ref}$), obtained on a pixel of the row or of the column.

15. Method according to either one of Claims 13 and 14, wherein the sensor comprises different masked pixels ($32_i$), each masked pixel being positioned between at least two open pixels ($31_i$, $31_{i+1}$) along the same row and two open pixels ($31_k$, $31_{k+1}$) along the same column, steps a) to f) being carried out on each masked pixel ($32_i$) as well as on the open pixels ($31_i$, $31_{i+1}$, $31_k$, $31_{k+1}$) between which the masked pixel is positioned, step g) being carried out on the basis of a phase difference determined on the basis of an intensity ($I'_i$) of a light wave detected by each masked pixel.

**Fig. 1A**

**Fig. 1B**

**Fig. 1C**

$$\varphi_{i+1} - \varphi_i$$

**Fig. 1D**

**Fig. 2A**

**Fig. 2B**

EP 3 503 193 B1

**Fig. 2C**

25

X

Z

30

**Fig. 3A**

24

**Fig. 3B**

$24_i$

**Fig. 3C** $32_i$

SiO₂

**Fig. 3D**

Al

SiN
27

**Fig. 3E**

22b

**Fig. 3F**

SiN

**Fig. 3G**

21b   22b

**Fig. 3H**

31ᵢ   32ᵢ

21a   22a   21ᵢ   22ᵢ

31ᵢ   32ᵢ

**Fig. 3I**

28

**Fig. 3J**   32ᵢ

Al

**Fig. 3K**   32ᵢ

25ᵢ

**Fig. 3L**   32ᵢ

Fig. 4A

Fig. 4B

Fig. 4C

Fig. 4D

Fig. 4E

**Fig. 4F**

**Fig. 4G**

**Fig. 4H**

**Fig. 4I**

**Fig. 4J**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2016064436 A
- WO 2008090330 A
- US 20120218379 A
- WO 2017162985 A
- WO 2016189257 A
- EP 3147646 A
- US 2017317125 A

**Littérature non-brevet citée dans la description**

- **TAILLAERT D.** Compact efficient broadband grating coupler for silicon-on-insulator waveguides. *Optics Letters,* 01 Décembre 2004, vol. 29 (23